(19) **Europäisches Patentamt / European Patent Office / Office européen des brevets**

(11) **EP 1 844 773 A1**

(12) # EUROPEAN PATENT APPLICATION
published in accordance with Art. 158(3) EPC

(43) Date of publication:
**17.10.2007 Bulletin 2007/42**

(21) Application number: **06712902.3**

(22) Date of filing: **02.02.2006**

(51) Int Cl.:
*A61K 31/4164* (2006.01)  *A61K 9/70* (2006.01)
*A61K 47/06* (2006.01)  *A61K 47/10* (2006.01)
*A61K 47/12* (2006.01)  *A61K 47/14* (2006.01)
*A61K 47/16* (2006.01)  *A61K 47/22* (2006.01)
*A61K 47/32* (2006.01)  *A61K 47/46* (2006.01)
*A61P 1/00* (2006.01)  *A61P 11/00* (2006.01)
*A61P 11/06* (2006.01)  *A61P 13/02* (2006.01)

(86) International application number:
**PCT/JP2006/301759**

(87) International publication number:
**WO 2006/082888 (10.08.2006 Gazette 2006/32)**

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **03.02.2005 JP 2005027826**

(71) Applicants:
- **KYORIN PHARMACEUTICAL CO., LTD.
  Tokyo 101-8311 (JP)**
- **ONO PHARMACEUTICAL CO., LTD.
  Osaka-shi, Osaka 541-8526 (JP)**

(72) Inventors:
- **TAKAHASHI, Koichi
  Osaka, 581080 (JP)**
- **SAKAI, Yoshiki
  home, Shimamoto-cho, Mishima-gun, Osaka,
  (JP)**

(74) Representative: **Henkel, Feiler & Hänzel
Patentanwälte
Maximiliansplatz 21
80333 München (DE)**

(54) **PERCUTANEOUS ABSORPTION PREPARATION**

(57)  This present invention provides a percutaneous absorption type pharmaceutical preparation which comprises 4-(2-methyl-1-imidazolyl)-2,2-diphenylbutylamide as the active ingredient and a backbone for transdermal system, and satisfies at least one of the following conditions (1) and (2). The pharmaceutical preparation of the present invention enables absorption of 4-(2-methyl-1-imidazolyl)-2,2-diphenylbutylamide, which has a low ability to be absorbed from the skin, from the skin into the body continuously and efficiently.
(1) The active ingredient content in one preparation or one time administration preparation is from about 0.1 mg to about 10 mg,
(2) the size is from about 1 cm$^2$ to about 300 cm$^2$.

EP 1 844 773 A1

**Description**

Technical Field

The present invention relates to a percutaneous absorption type pharmaceutical preparation, which comprises 4-(2-methyl-1-imidazolyl)-2,2-diphenylbutylamide (to be referred to as imidafenacin hereinafter) as the active ingredient.

Background of the Invention

Imidafenacin is a compound having a selective M3 muscarine receptor antagonism (JP-A-7-215943; Patent Reference 1), which is promising as an agent for treating pollakiurea and urinary incontinence accompanied by overactive bladder (Bioorg. Med. Chem., 1999, vol. 7, pp. 1151 - 1161; Non Patent Reference 1).

When imidafenacin is clinically applied, an oral solid preparation which comprises imidafenacin has been proposed, similar to the dosage form of the agent for treating pollakiurea and urinary incontinence which is already on the market (International Publication No. 01/34147; Patent Reference 2).

In recent years, aging of population has been progressed who is accompanied by the high degree advancement of medical treatment and exerting various influences upon the society. For example, it is said that about half the number of bedridden, dementia and the like old people who require care have overactive bladder, which cause pollakiurea and urinary incontinence. It is defined that the overactive bladder is symptomatic syndrome, which generally means a condition with a feeling of urinary tenesmus, which accompanies pollakiurea and nocturia, wherein the presence or absence of urge incontinence does not matter. Also, the urinary incontinence is a state of involuntarily leaking urine, which is defined as a symptom that accompanies social and hygienic problems. The urinary incontinence is mainly classified into urge incontinence, stress incontinence, functional incontinence and overflow incontinence, and the urge incontinence increases with aging. Additionally, it is expected that the number of pollakiurea and urinary incontinence patients will increase and changes in the class of the patients will occur in the future. For example, pollakiurea and urinary incontinence occur frequently in long-term bedridden patients of cerebrovascular disorder such as cerebral infarction and intracerebral bleeding, or after a cerebrospinal injury or various tumor operations. Also, these aged people are in a dysphagia state in many cases, and the oral preparation, which is taken at a certain interval is difficult to apply in many cases. Additionally, since oral administration increases amount of agent exposure to the liver and also increases its maximum blood concentration, the side effect expressing ratio is increased. Accordingly, from the viewpoint of making qualitative improvement of life of patients who are difficult to undergo oral administration, a non-oral type pharmaceutical preparation having further high directivity to patients is in desired.

On the other hand, based on the idea of a system for delivering a drug aimed at optimizing treatments, namely drug delivery system, a percutaneous absorption type pharmaceutical preparation having a function which draws a line from the conventional external preparations, namely a function to maintain effects of agents and reduce side effects thereof, have been drawn attention and a large number of studies have been carried out. Specifically, a non-oral type administration route, particularly a transdermal administration system (transdermal drug delivery system) represented by ointments and Plaster and Pressure Sensitive Adhesives, draws attention as effective drug delivery route. In the transdermal administration, since an agent directly enters subcutaneous capillary vessel, there is no liver-first-pass effect. Additionally, since it reaches the object region almost without its metabolism and degradation, bioavailability of the agent becomes high. Additionally, its stable pharmacokinetics in blood is obtained even in old people who have large individual differences in the liver function.

However, since percutaneous absorption ability of agents is generally poor due to the barrier function of the skin, it is difficult in many cases to deriver necessary amount of an agent for expressing its drug effect with a practical but limited sticking area. Additionally, it is well known that absorption of a hydrophilic drug or a drug in the form of a salt from the skin is difficult to attain since it has large polarity by itself. The greatest barrier in the percutaneous absorption of an agent is the keratin layer, which is a complex structure of small keratinized cell residues separated by the extracellular lipid regions, which has far lower drug permeability in comparison with the mouth or stomach mucous membrane. Additionally, percutaneous absorption agents have many problems such as stability, persistence, effect, safety (expression of side effects), adhesiveness, sense of incongruity, skin irritation (erythema, edema, itchiness, eruption, pigmentation or the like) and the like of drugs.

[Patent Reference 1] JP-A-7-215943

[Patent Reference 2] International Publication No. 01134147

[Non-patent Reference 1] Bioorg. Med. Chem., 1999, vol. 7, pp. 1151 - 1161

Disclosure of the Invention

Problems to be Solved by the Invention

The oral muscarine receptor antagonist used as an agent for treating pollakiurea and urinary incontinence generally expresses dry mouth, feeling of residual urine, difficulty of urination, constipation, diarrhea, urinary retention, unpleasant feeling in the stomach, abdominal pain, abnormal feeling in the eye (mydriasis), vertigo and the like as its side effects which are clinically serious problems. With the aim of improving these, a selective M3 muscarine receptor antagonist imidafenacin was found and has been examined on the pollakiurea and urinary incontinence of overactive bladder patients. In general, a percutaneous absorption type administration agent, from which stable pharmacokinetics in blood can be easily obtained and in which administration can be immediately stopped by peeling it off, is safer than the oral administration by which cannot avoid temporary increase of blood concentration. However, since absorption of imidafenacin from the skin is very low, it is difficult to ensure the blood concentration sufficient for its efficacy expression by absorption from the skin for a prolonged period of time.

The object of the present invention is for solving the aforementioned problems and provision of a percutaneous absorption type pharmaceutical preparation, which can effect absorption of imidafenacin from the skin for a prolonged period of time by increasing skin permeation of imidafenacin. Means for Solving the Problems

To achieve the aforementioned object, inventors of the present invention have carried out intensive studies and found as a result that a percutaneous absorption type pharmaceutical preparation which uses 4-(2-methyl-1-imidazolyl)-2,2-diphenylbutylamide as the active ingredient; comprises a backbone for transdermal system; and satisfies at least one condition of the following conditions:

> (1) the active ingredient content in one preparation or one time administration preparation is from about 0.1 mg to about 10 mg,
> (2) its size is from about 1 cm$^2$ to about 300 cm$^2$; can achieve the object. Thus, the present invention was accomplished.

Namely, the present invention relates to a percutaneous absorption type pharmaceutical preparation consisting of the following constructions.

> [1] A percutaneous absorption type pharmaceutical preparation which comprises 4-(2-methyl-1-imidazolyl)-2,2-diphenylbutylamide as the active ingredient and a backbone for transdermal system, and satisfies at least one of the following conditions (1) and (2):

> > (1) the active ingredient content in one preparation or one time administration preparation is from about 0.1 mg to about 10 mg,
> > (2) the size is from about 1 cm$^2$ to about 300 cm$^2$.

> [2] The pharmaceutical preparation according to [1], wherein the backbone for transdermal system comprises (i) one or more adhesive(s) selected from a styrene-isoprene-styrene block copolymer, a silicone rubber, a polyisobutylene rubber, a rosin resin, an polyacrylate and an alicyclic saturated hydrocarbon resin and (ii) an amphipathic solubilizing agent, a percutaneous permeation accelerator and/or a skin irritation alleviating agent.
> [3] The pharmaceutical preparation according to [2], wherein the backbone for transdermal system consists of (i) one or more adhesive(s) selected from a styrene-isoprene-styrene block copolymer, a silicone rubber, a polyisobutylene rubber, a rosin resin and an alicyclic saturated hydrocarbon resin, (ii) an amphipathic solubilizing agent and (iii) a percutaneous permeation accelerator.
> [4] The pharmaceutical preparation according to [2], wherein (i) the adhesive is one or more adhesive(s) selected from a styrene-isoprene-styrene block copolymer, a silicone rubber, an polyacrylate, a polyisobutylene rubber, a rosin resin and an alicyclic saturated hydrocarbon resin and (ii) the amphipathic solubilizing agent is one or more agent(s) selected from N-methyl-2-pyrrolidone, isopropyl myristate, propylene glycol, triacetin, benzyl alcohol, oleyl alcohol, oleic acid, diethyl sebacate, diisopropyl adipate, liquid paraffin and cetyl lactate, the percutaneous permeation accelerator is one or more accelerator(s) selected from triacetin, Crotamiton, cetyl lactate, diisopropyl adipate, oleic acid and oleyl alcohol, and the skin irritation alleviating agent is Crotamiton.
> [5] The pharmaceutical preparation according to [4], wherein the adhesive is (i) a combination of a styrene-isoprene-styrene block copolymer and a rosin resin; (ii) a silicone rubber; (iii) a combination of a silicone rubber and a rosin resin; (iv) a combination of a silicone rubber, a rosin resin and an alicyclic saturated hydrocarbon resin; (v) an polyacrylate; (vi) a combination of an polyacrylate and a silicone rubber; or (vii) a combination of a styrene-isoprene-styrene block copolymer, a polyisobutylene rubber, a rosin resin and an alicyclic saturated hydrocarbon resin.

[6] The pharmaceutical preparation according to [2], wherein (i) the adhesive is a combination of a styrene-isoprene-styrene block copolymer and a rosin resin, and (ii-1) the amphipathic solubilizing agent is N-methyl-2-pyrrolidone, propylene glycol, triacetin, oleyl alcohol, oleic acid or cetyl lactate; (ii-2) the percutaneous permeation accelerator is triacetin, Crotamiton, cetyl lactate, oleic acid or oleyl alcohol; or (ii-3) the combination of an amphipathic solubilizing agent and a percutaneous permeation accelerator is oleic acid and Crotamiton, oleyl alcohol and Crotamiton, cetyl lactate and Crotamiton or triacetin and Crotamiton.

[7] The pharmaceutical preparation according to [2], wherein (i) the adhesive is a silicone rubber, and (ii-1) the amphipathic solubilizing agent is N-methyl-2-pyrrolidone, isopropyl myristate, propylene glycol, triacetin, oleyl alcohol, oleic acid or cetyl lactate; (ii-2) the percutaneous permeation accelerator is triacetin, Crotamiton, cetyl lactate, oleic acid or oleyl alcohol; or (ii-3) the combination of an amphipathic solubilizing agent and a percutaneous permeation accelerator is oleic acid and Crotamiton or oleyl alcohol and Crotamiton.

[8] The pharmaceutical preparation according to [2], wherein (i) the adhesive is a combination of a styrene-isoprene-styrene block copolymer, a polyisobutylene rubber, a rosin resin and an alicyclic saturated hydrocarbon resin, and (ii-1) the amphipathic solubilizing agent is one or more agent(s) selected from N-methyl-2-pyrrolidone, isopropyl myristate, propylene glycol, triacetin, oleyl alcohol, oleic acid, liquid paraffin and cetyl lactate; the percutaneous permeation accelerator is at least one or more accelerator(s) selected from triacetin, Crotamiton, cetyl lactate, oleic acid and oleyl alcohol; and the skin irritation alleviating agent is Crotamiton.

[9] The pharmaceutical preparation according to [2], wherein based on 1 part by mass of the active ingredient, the adhesive is from about 25 parts by mass to about 350 parts by mass; and (i) the amphipathic solubilizing agent and/or percutaneous permeation accelerator is from about 2 parts by mass to about 400 parts by mass or (ii) the amphipathic solubilizing agent and/or percutaneous permeation accelerator is from about 2 parts by mass to about 200 parts by mass; and the skin irritation alleviating agent is from about 0.1 part by mass to about 10 parts by mass.

[10] The pharmaceutical preparation according to [3], wherein the amphipathic solubilizing agent is (i) liquid paraffin and (ii) N-methyl-2-pyrrolidone and/or propylene glycol, and the percutaneous permeation accelerator is oleic acid.

[11] The pharmaceutical preparation described in [3], wherein based on 1 part by mass of the active ingredient, the adhesive is from about 25 parts by mass to about 350 parts by mass; the amphipathic solubilizing agent is from about 2 parts by mass to about 400 parts by mass; and the percutaneous permeation accelerator is from about 2 parts by mass to about 200 parts by mass.

[12] The pharmaceutical preparation according to any one of [2] to [11], which further comprises from about 0.01 part by weight to about 10 parts by weight of dibutylhydroxytoluene or DL-$\alpha$-tocopherol, based on 1 part by weight of the active ingredient.

[13] The pharmaceutical preparation according to [1], which comprises 4-(2-methyl-1-imidazolyl)-2,2-diphenylbutylamide, which is a soluble type or a mixed type of soluble type and non-soluble type as the active ingredient.

[14] The pharmaceutical preparation according to any one of [1] to [13], wherein blood concentration of 4-(2-methyl-1-imidazolyl)-2,2-diphenylbutylamide is maintained in a range of from about 10 pg/ml to about 10 ng/ml for about 0.5 day to about 2 days after its administration.

[15] The pharmaceutical preparation according to [1], which is a Plaster and Pressure Sensitive Adhesive.

[16] The pharmaceutical preparation according to [15], which has an adhesive plaster having a thickness of from about 10 $\mu$m to about 2000 $\mu$m

[17] The pharmaceutical preparation according to [15], which is a plaster or a cataplasm.

[18] The pharmaceutical preparation according to [1], which is an agent for preventing and/or treating a disease selected from pollakiurea and urinary incontinence accompanied by over active bladder, asthma, chronic obstructive pulmonary disease and irritable bowel syndrome.

[19] A Plaster and Pressure Sensitive Adhesive which uses 4-(2-methyl-1-imidazolyl)-2,2-diphenylbutylamide as an active ingredient, which comprises (i) an adhesive consisting of a combination of a styrene-isoprene-styrene block copolymer, a polyisobutylene rubber, a rosin resin and an alicyclic saturated hydrocarbon resin, which is from about 25 parts by mass to about 160 parts by mass, based on 1 part by mass of the active ingredient; (ii) liquid paraffin which is from about 20 parts by mass to about 80 parts by mass, based on 1 part by mass of the active ingredient; (iii) N-methyl-2-pyrrolidone and/or propylene glycol which is from about 2 parts by mass to about 100 parts by mass, based on 1 part by mass of the active ingredient; and (iv) oleic acid which is from about 2 parts by mass to about 50 parts by mass, based on 1 part by mass of the active ingredient, and which comprises (v) can maintain blood concentration of the active ingredient within a range of from about 10 pg/ml to about 3 ng/ml for about 0.5 day to about 2 days after its administration, wherein it satisfies at least one of the following conditions (1) to (3):

(1) the active ingredient content in one preparation or one time administration preparation is from about 0.1 mg to about 2 mg,
(2) the size is from about 1 cm$^2$ to about 40 cm$^2$,
(3) thickness of the adhesive plaster is from about 20 $\mu$m to about 200 $\mu$m.

Effect of the Invention

According to the present invention, imidafenacin, which has low absorption property from the skin can be stably absorbed from the skin into the body persistently and efficiently.

Best Mode for Carrying Out the Invention

The imidafenacin as the active ingredient of the percutaneous absorption type pharmaceutical preparation of the present invention is 4-(2-methyl-1-imidazolyl)-2,2-diphenylbutylamide. For example, 4-(2-methyl-1-imidazolyl)-2,2-diphenylbutylamide can be produced by the method described in Example 11 of JP-A-7-215943. According to the present invention, imidafenacin can be used as both of its free form and a medically acceptable salt thereof. Examples of the medically acceptable salt include an inorganic acid (e.g., hydrochloric acid, sulfuric acid, hydrobromic acid or the like) and an organic acid (e.g., maleic acid, fumaric acid, acetic acid, oxalic acid, tartaric acid, benzenesulfonic acid or the like). Additionally, the percutaneous absorption type pharmaceutical preparation of the present invention can also be used as percutaneous absorption type pharmaceutical preparations of similar selective M3 muscarine antagonists: for example, tolterodine, darifenacin, solifenacin and the like.

As the backbone for transdermal system in the percutaneous absorption type pharmaceutical preparation which is used in the present invention, bases selected from the group consisting of amphiphilic solubilizing agents, suspending agents, softening agents, emulsifying agents, buffer agents, percutaneous permeation accelerators, adhesives, adhesion reinforcing agents, binders, skin irritation alleviating agents and additives can be used alone or as a combination of two or more species. Adhesives, and a single species or a combination of two or more species of bases selected from the group consisting of amphiphilic solubilizing agents, suspending agents, softening agents, emulsifying agents, buffer agents, percutaneous permeation accelerators, adhesion reinforcing agents, binders, skin irritation alleviating agents and additives are preferable.

Examples of the adhesives or adhesion reinforcing agents include natural rubber, crude rubber, high molecular weight rubber, RSS No.1 crude rubber, styrene isoprene rubber, styrene-butadiene rubber (SBR), cis-polyisoprene rubber, polyisobutylene rubber (high molecular weight polyisobutylene, low molecular weight polyisobutylene or a mixture thereof), high cis-polyisoprene rubber, styrene-isoprene-styrene block copolymer (SIS), styrene-isobutylene-styrene block copolymer, styrene-butadiene-styrene block copolymer (SBS), silicone rubber, silicone rubber, methyl vinyl ether-maleic anhydride copolymer, polyacrylate ((meth)acrylic acid-(meth)acrylic acid ester copolymer, acrylic acid-acrylic acid octyl ester copolymer, acrylic acid ester-vinyl acetate copolymer, 2-ethylhexyl acrylate-vinyl pyrrolidone copolymer solution, 2-ethylhexyl acrylate-2-ethylhexyl methacrylate-dodecyl methacrylate copolymer solution, methyl acrylate-2-ethylhexyl acrylate copolymer resin emulsion, methacrylic acid-n-butyl acrylate copolymer, silk fibroin acrylate copolymer, acryl resin alkanolamine solution or the like), polybutene, maleinated rosin glycerol ester, alicyclic saturated hydrocarbon resin (Alcon or the like), aliphatic hydrocarbon resin, petroleum resin (Quintone, Escolets or the like), terpene resin, higher aqueous high polymer (starch acrylate or the like), hydrophilic high polymer (polyacrylic acid, polyacrylic acid aqueous solution, sodium polyacrylate, polyacrylic acid partial neutralization product, carboxy vinyl polymer, methyl cellulose, carboxymethylcellulose (CMC), carboxymethylcellulose sodium (CMC Na), hydroxyethyl cellulose, hydroxypropyl cellulose, hydroxypropylmethyl cellulose (HPMC) (hydroxypropylmethyl cellulose 2208, hydroxypropylmethyl cellulose 2906, hydroxypropylmethyl cellulose 2910 or the like), macrogol 400, macrogol 6000, polyvinyl alcohol (PVA), polyvinyl pyrrolidone (PVP), sodium alginate, alginic acid propylene glycol ester, pectin, xanthangum, xanthan gum, locust bean gum, guar gum, arabinogalactan, sodium hyaluronate or the like), rosin resin (rosin, ultra hypochromic system rosin derivative, hydrogenated rosin glycerol ester or the like), propylene glycol, dibutylhydroxytoluene, glycerol, glucono-δ-lactone, gum arabic, gum arabic powder, ester gum, dammar rubber, hydrated lanolin, gelatin, dextrin, casein sodium, collodion, tragacanth, tragacanth powder, wheat starch, white vaseline, vaseline-lanolin alcohol mixture, carnauba wax, starch syrup, magnesium aluminum silicate, light silicic anhydride, corn oil, castor oil, mixture of sulfonated castor oil potassium salt and alkylbenzene sulfonate, benzyl acetate, talc, polyisobutylene, polyterpene system resin, coumarone-indene resin, terpene-phenyl resin, xylene resin and the like.

Examples of the amphiphilic solubilizing agents include N-methyl-2-pyrrolidone, L-menthol, methyl ethyl ketone, methyl isobutyl ketone, higher fatty acid ester (isopropyl myristate (IPM), isopropyl palmitate, oleyl oleate or the like), lauric acid diethanolamide, triethyl citrate, dimethylimidazolidinone, glycerol fatty acid ester (lipophilic glycerol monooleate or the like), polyglycerol fatty acid ester, sorbitan fatty acid ester (sorbitan monooleate, sorbitan trioleate, sorbitan sesquioleate or the like), polyoxyethylene sorbitan ester, polyoxyethylene sorbitol fatty acid ester (Polysorbate 20, Polysorbate 60, Polysorbate 80, polyoxyethylene sorbitan monolaurate or the like), polyoxyethylene alkyl formaldehyde condensation product, polyoxyethylene sterol-hydrogenated sterol, polyoxyethylene glycol fatty acid ester, polyoxyethylene lanoline, sodium phosphate polyoxyethylene lauryl ether, polyoxyethylene alkyl ether (Lauromacrogol or the like), polyoxyethylene polyoxypropylene alkyl ether, polyoxypropylene glycol 2000, polyoxyethylene polyoxypropylene glycol (polyoxyethylene(160) polyoxypropylene(30) glycol, polyoxyethylene(196) polyoxypropylene(67) glycol or the like), polyoxyethylene alkyl phenyl ether (polyoxyethylene nonyl phenyl ether or the like), polyvinyl alcohol, beeswax derivative, polyoxyethylene alkyl amine-fatty acid amide, polyoxyethylene alkyl ether phosphate-phosphoric acid salt, monofatty acid polyoxyethylene hydrogenated castor oil, polyoxyethylene castor oil, polyoxyethylene hydrogenated castor oil, polyoxyethylene hydrogenated castor oil 10, polyoxyethylene hydrogenated castor oil 40, polyoxyethylene hydrogenated castor oil 50, polyoxyethylene hydrogenated castor oil 60, polyethylene glycol monooleate, polyethylene glycol monostearate, ethoxy glycol, propylene glycol, 1,3-butylene glycol, polyethylene glycol 400, methanol, acetone, ethyl acetate, ethyl lactate, triacetin, pantothenyl ethyl ether, ethylene glycol monobutyl ether, dimethyl ether, diethyl ether, monoethanolamine, diethanolamine, diethylamine, isopropanolamine, diisopropanolamine, triethanolamine, 2-amino-2-methyl-1-propanol, 2-amino-2-methyl-1-propanediol, N,N-dimethylacetamide, dimethyl sulfoxide, dodecyl sulfoxide, geraniol denatured alcohol, 8-acetylsucrose denatured alcohol, linalyl acetate denatured alcohol, phenylethyl alcohol denatured alcohol, benzyl alcohol, butanol, 2-butanol, glycerol, higher alcohol (lauryl alcohol, isopropanol, isostearyl alcohol, octyl dodecanol, oleyl alcohol or the like), geraniol, diethylene glycol, ethoxy diglycol, diisopropylene glycol, propylene glycol fatty acid ester, propylene glycol monocaprate (S-218), propylene carbonate, thioglycolic acid, propionic acid, methanesulfonic acid, glacial acetic acid, lactic acid, butyric acid, citric acid, hydrochloric acid, phosphoric acid, sodium hydrogenphosphate, potassium dihydrogenphosphate, potassium iodide, fatty acid (capric acid, adipic acid, sebacic acid, myristic acid, oleic acid or the like), ichthammol, benzyl benzoate, nicotinic acid amide, nicotinic acid benzyl ester, dibasic acid diesters (diethyl sebacate, diisopropyl sebacate, diisopropyl adipate and the like), rapeseed oil, soybean oil, soybean lecithin, D-mannitol, zinc sulfate, aluminum sulfate, sodium thiosulfate, middle chain fatty acid triglyceride, β-cyclodextrin, liquid paraffin, cetyl lactate, octyldodecyl lactate and the like.

Examples of the suspending agents include ethanol, stearyl alcohol, cetanol, polyhydric alcohol (ethylene glycol, propylene glycol, butanediol, triethylene glycol, polyethylene glycol (macrogol 200, macrogol 300, macrogol 400, macrogol 4000, macrogol 600, macrogol 1500 or the like), cetomacrogol 1000, glycerol, ethylene glycol monostearate or the like), higher fatty acid esters (hexyl laurate, butyl stearate, isopropyl palmitate, isopropyl myristate, octyldodecyl myristate, myristyl myristate, glycerol fatty acid ester, glycerol monostearate, sorbitan fatty acid ester and the like), sorbitan monolaurate, polyoxyethylene sorbitol monolaurate, sorbitan trioleate, propylene glycol fatty acid ester, polyoxyethylene sorbitol fatty acid ester (Polysorbate 20, Polysorbate 60, Polysorbate 80 or the like), sorbitan sesquioleate, polyoxyethylene nonyl phenyl ether, polyoxyethylene hydrogenated castor oil, polyoxyethylene hydrogenated castor oil 60, polyoxyethylene(160) polyoxypropylene(30) glycol, macrogol 6000, dioctyl sodium sulfosuccinate, dimethyl polysiloxane-silicon dioxide mixture, carboxyvinyl polymer, methyl cellulose, hydroxypropyl cellulose, carboxymethylcellulose, carboxymethylcellulose sodium, crystalline cellulose-carmellose sodium, povidone, methylene-β-naphthalene sulfonate sodium, sodium erythorbate, liquid hydrocarbons (liquid paraffin and the like), animal and plant oils (almond oil, camellia oil, persic oil, mink oil, safflower oil, coconut oil, eucalyptus oil, soybean oil, sesame oil, corn oil, rapeseed oil, sunflower oil, cotton seed oil, olive oil, castor oil, peanut oil, wheat germ oil and the like), soybean lecithin, beeswax, white wax, hydrogenated oil, squalane, squalene, middle chain fatty acid triglyceride, gum arabic, gum arabic powder, xanthan gum, pectin, bentonite, sodium alginate, alginic acid propylene glycol ester, sodium chloride, benzalkonium chloride, kaolin, carrageenan, carnauba wax, dry aluminum hydroxide gel, magnesium aluminum silicate, aluminum magnesium silicate, potassium hydroxide, sodium hydroxide, aluminum stearate, phosphoric acid and the like.

Examples of the softening agents include allantoin, almond oil, olive oil, rapeseed oil, castor oil, cotton seed oil-soybean oil mixture, process oil, beef tallow, propylene glycol, polybutene, glycerol, liquid paraffin, light liquid paraffin, crystallized cellulose, macrogol 1500, squalane, squalene, purified lanolin, middle chain fatty acid triglyceride, glycerol monostearate, isopropyl myristate, crude rubber, ethyl lactate, cetyl lactate, octyldodecyl lactate, vanillylamide nonylate and high cis-polyisobutylene rubber.

Examples of the emulsifying agents include glycerol fatty acid ester (glycerol monooleate, glycerol lipophilic monooleate, glycerol monostearate, glycerol lipophilic monostearate, glycerol self emulsification type monostearate, polyoxyethyleneglyceryl triisostearate, glycerol monooleate-glycerol dioleate-propylene glycol mixed emulsifier or the like), sorbitan fatty acid ester, sorbitan monooleate, sorbitan trioleate, sorbitan monolaurate, sorbitan sesquioleate, sorbitan monostearate, sorbitan tristearate, sorbitan monopalmitate, glycerol fatty acid ester, propylene glycol fatty acid ester, polyglycerol fatty acid ester, polyoxyethylene glycerol fatty acid ester, polyoxyethylene glycerol monostearate, polyoxyethylene sorbitol fatty acid ester (polysorbate 20, polysorbate 40, polysorbate 60, polysorbate 65, polysorbate 80, polyoxyethylene sorbitan monolaurate, polyoxyethylene sorbitan monooleate, polyoxyethylene sorbitan trioleate, polyoxyethylene sorbitol tetraoleate or the like), cetomacrogol 1000, macrogol 20000, macrogol 300, macrogol 400, polyoxyethylene alkyl ether, polyoxyethylene cetyl ether, polyoxyethylene nonyl phenyl ether, polyoxyethylene octyl phenyl ether, polyoxyethylene oleyl ether, polyoxyethylene cetostearyl ether, polyoxyethylene sorbitol beeswax, polyoxyethylene oleyl ether sodium phosphate, polyoxyethylene cetyl ether sodium phosphate, polyoxyethylene lanolin, Lauromacrogol, polyoxyethylene glycol fatty acid ester, polyoxyethylene castor oil, polyoxyethylene hydrogenated castor oil, polyoxyethylene hydrogenated castor oil 5, polyoxyethylene hydrogenated castor oil 10, polyoxyethylene hydrogenated castor oil 20, polyoxyethylene hydrogenated castor oil 40, polyoxyethylene hydrogenated castor oil 50, polyoxyethylene hydrogenated castor oil 60, polyoxyethylene behenyl ether, α-monoisostearyl glyceryl ether, polyoxyethylene stearyl ether phosphate, polyoxyethylene(160) polyoxypropylene(30) glycol, polyoxyethylene(1) polyoxypropylene(1) cetyl ether, polyoxyethylene(10) polyoxypropylene(4) cetyl ether, polyoxyethylene(20) polyoxypropylene(4) cetyl ether, polyoxyethylene(20) polyoxypropylene(8) cetyl ether, propylene glycol, polyethylene glycol monooleate, ethylene glycol monostearate, polyethylene glycol monostearate, propylene glycol monostearate, polyethylene glycol distearate, polyethylene glycol monolaurate, lauric acid diethanolamide, fatty acid ester, isopropyl myristate, octyldodecyl myristate, sucrose fatty acid ester, carboxyvinyl polymer, methylcellulose, carboxymethyl cellulose sodium, hydroxypropyl cellulose, methyl acrylate-2-ethylhexyl acrylate copolymer resin emulsion, dioctyl sodium sulfosuccinate, liquid hydrocarbons (liquid paraffin, paraffin and the like), animal oil, plant oil, mineral oil, cotton seed oil-soybean oil mixture, egg yolk oil, egg yolk phospholipid, hydrocarbon, fatty acid (Stearic acid or the like), potassium stearate, sodium stearate, polyoxyl stearate 40, polyoxyl stearate 45, polyoxyl stearate 55, coconut oil fatty acid diethanolamide, higher alcohol silicone oil, beeswax, bleached beeswax, paraffin wax, spermaceti, squalane, squalene, carrageenan, potash soap, medicated soap, alkyldiaminoethylglycine hydrochloride liquid, reduced lanolin, sulfated castor oil potassium salt-alkylbenzene sulfonate mixture, diisopropanolamine, triethanolamine, diethyl sebacate, ethanol, glycerol, cetanol, myristyl alcohol, octyl dodecanol, oleyl alcohol, stearyl alcohol, cetostearyl alcohol, stearyl alcohol-polyoxyethylene stearyl ether mixture, cetanol-polyoxyethylene cetyl ether mixture wax, cetanol-polysorbate 60 mixture wax, cetanol-polyoxyethylene sorbitan monostearate mixture wax, cetostearyl alcohol- cetostearyl sodium sulfate mixture, pentaerystyl citrate higher fatty acid ester-beeswax-nonionic emulsifier mixture, dicetyl phosphate, sodium N-lauroyl-L-glutamate, tincture of benzoin, middle chain triglyceride, sodium N-acyl-L-glutamate, benzalkonium chloride, potassium hydroxide, sodium hydroxide, soybean lecithin, purified soybean lecithin, purified lanolin, talc, sodium cetyl sulfate, sodium lauryl sulfate, hydrogenated soybean phospholipid, partially hydrogenated soybean phospholipid, hydrogenated lanolin alcohol and pectin.

Examples of the buffer agents include citric acid, citric anhydride, sodium citrate, tartaric acid, succinic acid, dl-malic acid, fumaric acid, maleic acid, lactic acid, sodium lactate liquid, boric acid, borax, acetic acid, glacial acetic acid, sodium acetate, phosphoric acid, sodium hydrogenphosphate, potassium dihydrogenphosphate, sodium dihydrogenphosphate, anhydrous sodium hydrogenphosphate, anhydrous sodium dihydrogenphosphate, benzoic acid, sodium benzoate, primary, secondary or tertiary phosphate (sodium primary phosphate, sodium secondary phosphate, anhydrous trisodium phosphate, trisodium phosphate or the like), sodium metaphosphate, ε-aminocaproic acid, sodium hydroxide, sodium carbonate, sodium bicarbonate, ammonium chloride, sodium chloride, benzalkonium chloride, amino acid or a salt of the amino acid (glycine, L-arginine or the like), diethanolamine, diisopropanolamine, monoethanolamine, triethanolamine, triethanolamine hydrochloride, triethanolamine phosphoric acid ester sodium, chlorobutanol, glucose and rose oil.

Examples of the percutaneous permeation accelerators include triacetin (glyceryl triacetate), Crotamiton, urea, ethanol, decylmethyl sulfoxide, natural essential oil, terpene oil (peppermint oil, orange oil, terpin oil, L-menthol, d-limonene, menthone, pinene, piperitone, terpinene, terpinolene, terpinol, carveol or the like), fatty acid ester (glyceryl monolaurate, glyceryl monooleate, cetyl lactate, octyldodecyl lactate, glycerol monolaurate, glycerol monooleate, propylene glycol monolaurate, propylene glycol monooleate, sorbitan monolaurate, sorbitan monooleate or the like), dibasic acid diesters (diethyl cebacate, diisopropyl adipate), azacycloalkanes (Eizon, 1-(2-(decylthio)ethyl)azacyclopentan-2-on and the like), fatty acid or aliphatic alcohols (oleic acid, lauric acid, myristic acid, oleyl alcohol, isopropanol, lauryl alcohol and the like), polyoxyethylene(2) lauryl ether, polyoxyethylene(2) oleyl ether, lauryl diethanolamide, isopropyl myristate, N-hydroxymethyl lactate, sorbitol, squalene and the like.

Examples of the skin irritation alleviating agents include glycerol, Crotamiton, allantoin, antihistaminic agent (diphenhydramine or the like), anti-inflammatory agent (glycyrrhetinic acid or the like), and steroidal agent and the like.

Examples of the additional additive agents include sloppiness forming agent (gelatin or the like); powdery excipient (kaolin, bentonite, zinc oxide or the like); petroleum resin (Quinton, Alcon or the like); D-sorbitol; D-sorbitol liquid; sucrose; surfactant (polyoxyethylene hydrogenated castor oil 20, polyoxyethylene hydrogenated castor oil 60, polyoxyethylene sorbitol fatty acid ester (polysorbate 20, polysorbate 60, polysorbate 80, polyoxyethylene sorbitan monolaurate or the like), polyoxyethylene fatty acid ester (polyoxyl stearate 40 or the like), sorbitan fatty acid ester (sorbitan monooleate, sorbitan trioleate, sorbitan monolaurate, sorbitan sesquioleate or the like), glycerol self emulsification type monostearate, glycerol monostearate, sorbitan monostearate, sucrose fatty acid ester, Macrogol 400, Lauromacrogol, polyoxyethylene lauryl ether sodium phosphate, polyoxyethylene oleyl ether phosphate, polyoxyethylene nonyl phenyl ether, polyoxyethylene octyl phenyl ether, polyoxyethylene polyoxypropylene glycol (polyoxyethylene(120) polyoxypropylene(40) glycol, polyoxyethylene(160) polyoxypropylene(30) glycol, polyoxyethylene(20) polyoxypropylene (20) glycol or the like), polyoxyethylene polyoxypropylene decyl tetradecyl ether, alkyl allyl polyether alcohol, polyoxyethylene cetyl ether, polyoxyethylene oleylamine, polyoxyethylene sorbitol beeswax, diethanolamide laurate, stearyl alcohol, dibasic acid diesters (diethyl sebacate or the like), squalane, N-cocoyl-L-arginine ethyl ester DL-pyrrolidone carboxylic acid salt, N-cocoyl-N-methylaminoethyl sodium sulfonate, cetanol, cetomacrogol 1000, sodium lauryl sulfate or the like); antiseptics and/or antioxidants (parabens (methyl paraben and the like), sorbic acid or salts thereof, butylhydroxyanisole (BHA), dibutylhydroxytoluene (BHT), DL-α-tocopherol, ascorbic acid palmitate, nordihydroxyguaiarectic acid, guaiacol esters, 1,3-butylene glycol, sodium dihydroacetate, propyl gallate and the like), salt which form trivalent metal ion (aluminum chloride, alum, aluminum allantoate or the like); moisture keeping agent (alkaline earth metals (magnesium chloride and the like), urea, glycerol, sodium hyaluronate or the like); flavoring agent (peppermint oil, orange oil, chamomile oil, spearmint oil, clove oil, terpin oil, pine oil, peppermint liquid, Himalayan cedar oil, bergamot oil, eucalyptus oil, lavender oil, rose liquid, rose oil, Roman chamomile oil, Peru balsam, d-camphor, dl-camphor, d-borneol, dl-borneol, dl-menthol, l-menthol, geraniol, methyl salicylate, cinnamaldehyde, piperonal or the like); solvent (organic solvent (methanol, ethanol, ethyl acetate, n-butyl acetate, isopropanol, diethyl ether, 2-ethyl-1,3-hexanediol, methanol-modified alcohol, methyl isobutyl ketone, methyl ethyl ketone or the like), hydrochloric acid, water, physiological saline, aqueous ethanol, unsaturated fatty acid (oleic acid or the like), synthetic squalane, dipropylene glycol, ethylene glycol salicylate, petroleum benzine, trichloroethane, 8-acetyl sucrose-modified alcohol or the like).

Additionally, for example, a water-soluble high polymer compound (polyacrylic acid or a derivative thereof, a cellulose derivative, polyvinyl alcohol, gelatin, casein, polyethylene glycol, gum arabic, methyl vinyl ether/maleic anhydride copolymer, natural saccharides or the like), a fat-soluble high polymer compound (natural rubber, isoprene rubber, butyl rubber, styrene isobutylene block copolymer, styrene butadiene copolymer, silicone, lanolin, vaseline, plastibase, beeswax, whale oil, solid paraffin or the like), a fatty acid or a derivative thereof (a fatty acid having from 3 to 40 carbon atoms, a fatty acid ester thereof or a fatty acid alkali metal salt thereof), animal or plant oil and fat (olive oil, peppermint oil, soybean oil, cotton seed oil, corn oil, eucalyptus oil, castor oil, sesame oil, almond oil, camellia oil, persic oil, minke whale oil, safflower oil, coconut oil or the like), alcohols (alcohols having from 1 to 40 carbon atoms and also having from 1 to 10 hydroxyl groups in the molecule, such as ethanol, glycerol, propylene glycol, polyethylene glycol, octanediol, butanediol, D-sorbitol, benzyl alcohol and the like), a terpene compound (menthol, menthone, limonene, pinene, piperitone, terpinene, terpinolene, terpinol, carveol or the like), a surfactant (a nonionic surfactant (for example, polyoxyethylene sorbitan monooleate), an anionic surfactant, cationic surfactant) and water can also be used as the backbone for transdermal system, and these may be used alone or in combination of two or more species, or in combination with the aforementioned bases for external preparations.

Additionally, in order to obtain good transfer of the agent in percutaneous absorption type pharmaceutical preparations to the skin, particularly Plaster and Pressure Sensitive Adhesives, it is necessary to fix the Plaster and Pressure Sensitive Adhesives to the skin surface firmly. However, when the skin adhesiveness is too large, since their removal by peeling after use accompanies keratin peeling by the physical stimulation, skin stimulation occurs. Thus, there is an oil gel adhesion technique as a method for increasing skin adhesiveness of the Plaster and Pressure Sensitive Adhesives and reducing physical skin stimulation property. In the oil gel adhesion technique, an easily separable adsorbent, an adhesive which forms an adhesive layer for skin surface fixing use, a liquid component and the like are used. Examples of the easily separable adsorbent include a cross-linked acryl adhesive polymer, an ethylene-acetic acid-vinyl copolymer, an ethylene-ethyl acrylate copolymer or the like can be cited. Examples of the adhesive which forms an adhesive layer for skin surface fixing use include a method in which the adhesiveness is adjusted by blending a softening agent, an additive agent and the like based on a styrene-isoprene-styrene block copolymer (SIS) system, a natural rubber system, a synthetic rubber system, an acryl system, a silicone system, a vinyl ether system or the like can be cited. Since there is a case of adjusting the adhesion using a liquid component, a mineral oil, a fatty acid ester and the like are blended therein, as the softening agent.

With regard to the plaster preparations among the percutaneous absorption type pharmaceutical preparations of the present invention, in the method for testing adhesive strength of adhesive plaster by an Instron type tensile tester which is described in the japanese pharmacopoeia, the load is preferably from about 50 g to about 800 g, more preferably from about 50 g to about 400 g.

According to the present invention, the percutaneous absorption type pharmaceutical preparations are preferably Plaster and Pressure Sensitive Adhesives, and their examples include plaster preparations (e.g., an adhesive single layer type percutaneous absorption type pharmaceutical preparation, a reservoir type percutaneous absorption type pharmaceutical preparation and the like) and cataplasmas. According to the present invention, plaster preparations, particularly adhesive single layer type percutaneous absorption type pharmaceutical preparations, are desirable. The adhesive single layer type percutaneous absorption type pharmaceutical preparations are composed of imidafenacin which is an active ingredient and a percutaneous absorption type pharmaceutical preparation structure-forming body consisting of an "adhesive plaster" which has adhesiveness formed by combining a base selected from the aforementioned bases for external preparations, a "support" and a "release liner" which protects the adhesive plaster. According to the present invention, thickness of the adhesive plaster of the adhesive single layer type percutaneous absorption type pharmaceutical preparations is from about 10 μm to about 2000 μm. In order to withstand sticking to the skin for a long time and prevent adhesive transfer to the skin surface at the time of peeling removal, thickness of the adhesive plaster in the case of plaster preparations is preferably from about 10 μm to about 500 μm, more preferably from about 10 μm to about 200 μm, particularly from about 20 μm to about 200 μm. In the case of cataplasms, it is preferably from about 300 μm to about 2000 μm, more preferably from about 500 μm to about 1500 μm.

According to the present invention, the adhesive plaster consisting of imidafenacin as the active ingredient and a backbone for transdermal system consists of imidafenacin, an adhesive, and an amphipathic solubilizing agent, a suspending base, a softening agent, an emulsifying agent, a buffer agent, a percutaneous permeation accelerator, an adhesion reinforcing agent and/or a skin irritation alleviating agent. Preferably, it consists of imidafenacin, an adhesive, and an amphipathic solubilizing agent, a percutaneous permeation accelerator and/or a skin irritation-alleviating agent. More preferably, it consists of imidafenacin, an adhesive, an amphipathic solubilizing agent, and a percutaneous permeation accelerator.

As the adhesive, one species or a combination of two or more species selected from a styrene-isoprene-styrene block copolymer, a silicone rubber, a polyacrylate, a polyisobutylene rubber, a rosin resin and an alicyclic saturated hydrocarbon resin is particularly preferable. Specifically, (i) a combination of a styrene-isoprene-styrene block copolymer and a rosin resin, (ii) a silicone rubber, (iii) a combination of a silicone rubber and a rosin resin, (iv) a combination of a silicone rubber, a rosin resin and an alicyclic saturated hydrocarbon resin or (v) a combination of a styrene-isoprene-styrene block copolymer, a polyisobutylene rubber, a rosin resin and an alicyclic saturated hydrocarbon resin is preferable. Although the styrene-isoprene-styrene block copolymer is not particularly limited, SIS-5009, SIS-5229 and the like manufactured by Japan Synthetic Rubber Co., Ltd. are preferably used. Although the silicone rubber is not particularly limited, BIO-PSA Q7-4501 and the like manufactured by Dow-Corning are preferably used. Although the polyisobutylene rubber is not particularly limited, a high molecular weight polyisobutylene (preferably, Vistanex MML-100 manufactured by Exxon Chemical or the like) and/or a low molecular weight polyisobutylene (preferably, Oppanol B12SPN manufactured by BASF Japan or the like) is preferably used. Although the rosin resin is not particularly limited, but an ultra hypochromic rosin ester (ester gum) KE311 manufactured by Arakawa Chemical Industries Ltd. is preferably used. Although the alicyclic saturated hydrocarbon resin is not particularly limited, but Alcon P-100 manufactured by Arakawa Chemical Industries Ltd. is preferably used.

According to the present invention, the adhesive is preferably from about 20% by mass to about 99,9% by mass, more preferably from about 30% by mass to about 97% by mass, of the adhesive plaster mass. In the case of plaster preparations, the adhesive plaster mass is preferably from about 10 $g/m^2$ to about 500 $g/m^2$, more preferably from about 20 $g/m^2$ to about 200 $g/m^2$. In the case of cataplasmas, it is preferably from about 100 $g/m^2$ to about 2000 $g/m^2$, more preferably from about 500 $g/m^2$ to about 1500 $g/m^2$.

With regard to the blending amount of the adhesive, it is desirable to add from about 25 parts by mass to about 350 parts by mass, more desirable to add from about 25 parts by mass to about 160 parts by mass, based on 1 part by mass of imidafenacin.

Additionally, since the imidafenacin to be used in the present invention shows poor absorption property by the adhesive alone, it is preferable to add an amphipathic solubilizing agent, a percutaneous permeation accelerator and/or a skin irritation-alleviating agent to the adhesive. It is particularly preferable to add an amphipathic solubilizing agent and a percutaneous permeation accelerator to the adhesive.

As the amphipathic solubilizing agent, one species or two or more species selected from a higher fatty acid ester (isopropyl myristate, isopropyl palmitate, oleyl oleate or the like), a higher alcohol (lauryl alcohol, isopropanol, isostearyl alcohol, octyldodecanol, oleyl alcohol or the like), a fatty acid (capric acid, adipic acid, sebacic acid, myristic acid, oleic acid or the like), dibasic acid diesters (diethyl sebacate, diisopropyl sebacate, diisopropyl adipate and the like), triacetin, benzyl alcohol, cetyl lactate, octyldodecyl lactate and/or liquid paraffin are desirable, of which isopropyl myristate, oleyl alcohol, oleic acid, diethyl sebacate, diisopropyl adipate, triacetin, benzyl alcohol or liquid paraffin is particularly desirable.

As the amphipathic solubilizing agent, N-methyl-2-pyrrolidone, propylene glycol or a mixture of N-methyl-2-pyrrolidone and propylene glycol is also desirable.

As the amphipathic solubilizing agent, addition of (i) liquid paraffin and (ii) N-methyl-2-pyrralidone, propylene glycol or a mixture of N-methyl-2-pyrrolidone and propylene glycol is more desirable.

The blending amount of the amphipathic solubilizing agent to be blended is preferably from about 2 parts by mass to about 400 parts by mass, more preferably from about 2 parts by mass to about 100 parts by mass, based on 1 part by mass of imidafenacin.

The amount of liquid paraffin to be added as the amphipathic solubilizing agent is preferably from about 10 parts by mass to about 400 parts by mass thereof, more preferably from about 20 parts by mass to about 80 parts by mass, based on 1 part by mass of imidafenacin.

The amount of N-methyl-2-pyrrolidone to be added as the amphipathic solubilizing agent is preferably from about 2 parts by mass to about 100 parts by mass, and more preferably from about 3 parts by mass to about 20 parts by mass thereof, based on 1 part by mass of imidafenacin.

The amount of proylene glycol to be added as the amphipathic solubilizing agent is preferably from about 2 parts by mass to about 100 parts by mass, more preferably from about 3 parts by mass to about 20 parts by mass thereof, based on 1 part by mass of imidafenacin.

The amount of the mixture of N-methyl-2-pyrrolidone and propylene glycol to be added as the amphipathic solubilizing agent is preferably from about 2 parts by mass to about 100 parts by mass thereof, more preferably from about 3 parts by mass to about 20 parts by mass thereof, based on 1 part by mass of imidafenacin.

In this case, the ratio of N-methyl-2-pyrrolidone and propylene glycol is preferably from about 10:1 1 to about 1:10, more preferably from about 1:1 to about 1:3.

As the percutaneous permeation accelerator, triacetin, fatty acid or aliphatic alcohols (oleic acid, lauric acid, myristic acid, oleyl alcohol, isopropanol, lauryl alcohol and the like) and aliphatic ester (glyceryl monolaurate, glyceryl monooleate, cetyl lactate, octyldodecyl lactate, glycerol monolaurate, glycerol monooleate, propylene glycol monolaurate, propylene glycol monooleate, sorbitan monolaurate, sorbitan monooleate or the like) are preferable. Oleic acid, oleyl alcohol, triacetin and cetyl lactate are particularly preferable. Oleic acid is desirable in particularly preferable.

The amount of the percutaneous permeation accelerator to be blended is preferably from about 2 parts by mass to about 200 parts by mass, based on 1 part by mass of imidafenacin.

The amount of oleic acid to be added as the percutaneous permeation accelerator is preferably from about 2 parts by mass to about 50 parts by mass, more preferably from about 3 parts by mass to about 10 parts by mass, based on 1 part by mass of imidafenacin.

As the skin irritation alleviating agent, Crotamiton and a steroid agent are preferable.

Additionally, in order to effect absorption of imidafenacin from the skin for a long time, it is preferable that from about 25 parts by mass to about 350 parts by mass of the adhesive and from about 2 parts by mass to about 400 parts by mass of the amphipathic solubilizing agent and/or the percutaneous permeation accelerator are blended, or from about 2 parts by mass to about 200 parts by mass of the amphipathic solubilizing agent and/or the percutaneous permeation accelerator and from about 0.1 part by mass to about 10 parts by mass of the skin irritation alleviating agent are blended, based on 1 part by mass of imidafenacin.

An antiseptic and/or an antioxidant may be blended with the preparations of the present invention. As the antiseptic and/or antioxidant, dibutylhydroxytoluene (BTH) or DL-$\alpha$-tocopherol is preferable, and its blending amount is preferably from about 0.01 part by mass to about 10 parts by mass, based on I part by mass of imidafenacin.

According to the present invention, as a backbone for transdermal system of the Plaster and Pressure Sensitive Adhesives, (A) (i) a combination of a styrene-isoprene-styrene block copolymer and a rosin resin, (ii) a silicone rubber, (iii) a combination of a silicone rubber and a rosin resin, (iv) a combination of a silicone rubber, a rosin resin and an alicyclic saturated hydrocarbon resin, (v) an polyacrylate, (vi) a combination of an polyacrylate and a silicone rubber or (vii) a combination of a styrene-isoprene-styrene block copolymer, a polyisobutylene rubber, a rosin resin and an alicyclic saturated hydrocarbon resin, and (B) one species or two or more species of bases selected from isopropyl myristate, diethyl sebacate, diisopropyl adipate, liquid paraffin, oleic acid, oleyl alcohol, triacetin, benzyl alcohol, N-methyl-2-pyrrolidone, propylene glycol and cetyl lactate, or further (C) a base in which Crotamiton and BHT or DL-$\alpha$-tocopherol are combined are preferable.

Specifically, it is (A) a combination of a styrene-isoprene-styrene block copolymer with a rosin resin, and (B) diethyl sebacate, diisopropyl adipate, liquid paraffin, oleic acid, cetyl lactate, triacetin or isopropyl myristate with Crotamiton, oleic acid with Crotamiton, oleyl alcohol with Crotamiton, cetyl lactate with Crotamiton, N-methyl-2-pyrrolidone, propylene glycol, Crotamiton, or triacetin with Crotamiton, or (A) a silicone rubber, and (B) isopropyl myristate, diethyl sebacate, oleic acid, oleyl alcohol, oleic acid with Crotamiton, N-methyl-2-pyrralidane, propylene glycol, Crotamiton, or oleyl alcohol with Crotamiton, or (A) a combination of a silicone rubber with a rosin resin, and (B) isopropyl myristate, oleic acid, oleyl alcohol, isopropyl myristate with Crotamiton, oleic acid with Crotamiton, or oleyl alcohol with Crotamiton, or (A) a combination of a silicone rubber, a rosin resin and an alicyclic saturated hydrocarbon resin, and (B) isopropyl myristate, oleic acid, oleyl alcohol, isopropyl myristate with Crotamiton, oleic acid with Crotamiton, or oleyl alcohol with Crotamiton, or (A) a combination of a styrene-isoprene-styrene block copolymer, a polyisobutylene rubber, a rosin resin and an alicyclic saturated hydrocarbon resin, and (B) at least one base selected from isopropyl myristate, liquid paraffin, oleic acid, oleyl alcohol, cetyl lactate, triacetin, N-methyl-2-pyrrolidone, propylene glycol and Crotamiton. As the aforementioned combination of one or more species, liquid paraffin, isopropyl myristate, oleic acid, oleyl alcohol, cetyl lactate, triacetin, liquid paraffin with isopropyl myristate, liquid paraffin with oleic acid, liquid paraffin with oleyl alcohol, liquid paraffin with cetyl lactate, liquid paraffin with triacetin, liquid paraffin with Crotamiton, liquid paraffin with isopropyl myristate and Crotamiton, liquid paraffin with oleic acid and Crotamiton, liquid paraffin with oleyl alcohol and Crotamiton, liquid paraffin with cetyl lactate and Crotamiton, N-methyl-2-pyrrolidone, propylene glycol, N-methyl-2-pyrrolidone with propylene glycol, liquid paraffin with N-methyl-2-pyrrolidone, liquid paraffin with propylene glycol, liquid paraffin with N-methyl-2-pyrrolidone and propylene glycol, or liquid paraffin with triacetin and Crotamiton are preferable.

(A) a combination of a styrene-isoprene-styrene block copolymer with a rosin resin, and (B) oleic acid, cetyl lactate, triacetin, oleic acid with Crotamiton, oleyl alcohol with Crotamiton, cetyl lactate with Crotamiton, N-methyl-2-pyrrolidone, propylene glycol, Crotamiton, or triacetin with Crotamiton, or (A) a silicone rubber, and (B) oleic acid, oleyl alcohol, oleic acid with Crotamiton, N-methyl-2-pyrrolidone, propylene glycol, Crotamiton, or oleyl alcohol with Crotamiton, or (A) a combination of a silicone rubber with a rosin resin, and (B) isopropyl myristate, or isopropyl myristate with Crotamiton, or (A) a combination of a silicone rubber, a rosin resin and an alicyclic saturated hydrocarbon resin, and (B) oleyl alcohol, or oleyl alcohol with Crotamiton, or (A) a combination of a styrene-isoprene-styrene block copolymer, a polyisobutylene rubber, a rosin resin and an alicyclic saturated hydrocarbon resin, and (B) liquid paraffin with isopropyl myristate, liquid paraffin with oleic acid, liquid paraffin with oleyl alcohol, liquid paraffin with cetyl lactate, liquid paraffin with triacetin, liquid paraffin with Crotamiton, liquid paraffin with oleic acid and Crotamiton, liquid paraffin with oleyl alcohol and Crotamiton, liquid paraffin with cetyl lactate and Crotamiton, N-methyl-2-pyrrolidone, propylene glycol, N-methyl-2-pyrrolidone with propylene glycol, liquid paraffin with N-methyl-2-pyrrolidone, liquid paraffin with propylene glycol, liquid paraffin with N-methyl-2-pyrrolidone and propylene glycol, or liquid paraffin with triacetin and Crotamiton are more preferable.

According to the present invention, particularly preferable backbone for transdermal system is (i) (A) the adhesive is a combination of a styrene-isoprene-styrene block copolymer and a rosin resin, and (B) the amphipathic solubilizing agent, percutaneous permeation accelerator and/or skin irritation alleviating agent is oleic acid, cetyl lactate, triacetin, N-methyl-2-pyrrolidone, propylene glycol, Crotamiton, oleic acid with Crotamiton, oleyl alcohol with Crotamiton, cetyl lactate with Crotamiton or triacetin with Crotamiton, (ii) (A) the adhesive is a silicone rubber, and (B) the amphipathic solubilizing agent, percutaneous permeation accelerator and/or skin irritation alleviating agent is oleic acid, oleyl alcohol, N-methyl-2-pyrrolidone, propylene glycol, Crotamiton, oleic acid with Crotamiton, or oleyl alcohol with Crotamiton, or (iii) (A) the adhesive is a combination of a styrene-isoprene-styrene block copolymer, polyisobutylene, a rosin resin and an alicyclic saturated hydrocarbon resin, and (B) the amphipathic solubilizing agent, percutaneous permeation accelerator and/or skin irritation alleviating agent is one or more species selected from isopropyl myristate, liquid paraffin, oleic acid, oleyl alcohol, cetyl lactate, triacetin, N-methyl-2-pyrrolidone, propylene glycol and Crotamiton. As (iii) (B) the amphipathic solubilizing agent, percutaneous permeation accelerator and/or skin irritation alleviating agent, liquid paraffin, isopropyl myristate, oleic acid, oleyl alcohol, cetyl lactate, triacetin, Crotamiton, liquid paraffin with isopropyl myristate, liquid paraffin with oleic acid, liquid paraffin with oleyl alcohol, liquid paraffin with cetyl lactate, liquid paraffin with triacetin, liquid paraffin with Crotamiton, liquid paraffin with isopropyl myristate and Crotamiton, liquid paraffin with oleic acid and Crotamiton, liquid paraffin with oleyl alcohol and Crotamiton, liquid paraffin with cetyl lactate and Crotamiton, N-methyl-2-pyrrolidone, propylene glycol, N-methyl-2-pyrrolidane with propylene glycol, liquid paraffin with N-methyl-2-pyrrolidone, liquid paraffin with propylene glycol, liquid paraffin with N-methyl-2-pyrrolidone and propylene glycol, or liquid paraffin with triacetin and Crotamiton are preferable.

Although the support which constitutes structure-forming body of the adhesive single layer type percutaneous absorption type pharmaceutical preparation is not particularly limited, those which have a degree of flexibility that sense of incongruity is not markedly generated when it is stuck to the skin surface are desirable. For example, a single layer film consisting of a plastic film (polyethylene, polyethylene terephthalate, polyurethane, polyethylene, ethylene vinyl acetate, polypropylene, polyester, polyvinyl acetate, ethylene-vinyl acetate copolymer or the like), metal foil (aluminum foil or the like), non-woven fabric, cotton fabric, woven fabric, knit fabric, paper or the like, or a laminate film thereof can for example be used. The release liner is not particularly limited as long as it can be easily released in using the pharmaceutical preparations and can hold the adhesive plaster before covering of the release liner. For example, a sheet of paper, which is treated by a silicone resin or fluoride resin, a plastic film (polyethylene, polyethylene terephthalate, polyurethane, polyethylene, ethylene vinyl acetate, polypropylene, polyester, polyvinyl acetate, ethylene-vinyl acetate copolymer or the like) and the like can be used.

Preferable administration method of the present invention is a method in which it is re-stuck twice a day, once a day or once during 2 to 7 days, or applied before go to bed or before a necessary situation. It is a more preferable method in which it is re-stuck once a day or once during 2 to 4 days, or applied before going to bed or before a necessary situation.

Although the sticking region is not particularly limited, for example, it is the back of an ear, a brachial region, an abdominal region (preferably, the underbelly or the like), the chest, the back, the waist, the hip or the leg (preferably, medial femur, calf or the like) and the like, and 1 plaster or 2 to 4 plasters are applied to the aforementioned same region or different regions. When two or more plasters are stuck, although the positions are not particularly limited, it is desirable to apply to symmetrical positions. Additionally, in order to avoid skin irritation, it is desirable to not apply continuously to the same position.

The effective human blood concentration of imidafenacin in preventing and/or treating pollakiurea and urinary incontinence accompanied by over active bladder (OAB) or chronic obstructive pulmonary disease (COPD) can be extrapolated from the result of an animal test or the like.

According to a test, which used guinea pigs, blood concentration of imidafenacin to inhibit increase of intravesical pressure by choline stimulation at around its $ID_{50}$ value was about 1 ng/ml. Additionally, according to a test which used guinea pigs, the blood concentration at around its $ID_{50}$ value to inhibit airway contraction by choline stimulation was also about 1 ng/ml.

As a method for extrapolating the results obtained from guinea pigs to human, it is general to use values of from about 1/100 times to about 100 times of the value. Thus it is considered that its effective blood concentration in human is from about 10 pg/ml to about 100 ng/ml. Additionally, its blood concentration from which the intended effect can be obtained and at which side effects are not expressed can be rationally presumed to be from about 10 pg/ml to about 10 ng/ml from this value.

When the blood concentration exceeds 10 ng/ml, for example, dry mouth, feeling of residual urine, difficulty of urination, abnormal feeling in the eye, constipation, urinary retention and the like side effects are expressed.

The percutaneous absorption type pharmaceutical preparation containing imidafenacin, which is shown by the present invention is a pharmaceutical preparation which can maintain its blood concentration at from about 10 pg/ml to about 10 ng/ml in human.

The effective blood concentration in human is preferably from about 10 pg/ml to about 3 ng/ml, more preferably from about 100 pg/ml to about 3 ng/ml, further preferably from about 100 pg/ml to about 500 pg/ml. The range of said blood imidafenacin concentration is an optimum range, which expresses the object effect and does not express side effects.

With regard to the period for maintaining effective blood concentration in human, it is preferably from about 0.5 day to about 7 days, more preferably from about 0.5 day to about 4 days, further preferably from about 0.5 day to about 2 days.

According to the present invention, amount of imidafenacin to be blended with the percutaneous absorption type pharmaceutical preparation to maintain the aforementioned effective blood concentration in human varies on the administration period, the backbone for transdermal system to be used and blending amounts thereof. It is preferable that from about 0.01 mg to about 10 mg is formulated in one preparation or one time administration preparation. Particularly, in the case of a percutaneous absorption type pharmaceutical preparation which is sticked for 24 hours, it is preferable that from about 0.1 mg to about 1 mg is formulated in one preparation or one time administration preparation, and about 0.2 mg, about 0.3 mg, about 0.5 mg and about 1 mg are more preferable. Also, in the case of a percutaneous absorption type pharmaceutical preparation which is sticked for 48 hours, it is preferable that from about 0.2 mg to about 2 mg is formulated in one preparation or one time administration preparation, and about 0.2 mg, about 0.3 mg, about 0.4 mg, about 0.5 mg, about 0.7 mg, about 1 mg and about 2 mg are more desirable. Additionally, when the percutaneous absorption type pharmaceutical preparations of the present invention are Plaster and Pressure Sensitive Adhesives, the amount of imidafenacin is preferably from about 0,005% by mass to about 30% by mass, more preferably from about 0.05% by mass to about 10% by mass, based on the whole adhesive plaster.

According to the present invention, when the aforementioned percutaneous absorption type pharmaceutical preparations for maintaining the effective blood concentration in human are Plaster and Pressure Sensitive Adhesives, the size is preferably from about 1 cm$^2$ to about 300 cm$^2$, more preferably from about 1.5 cm$^2$ to about 280 cm$^2$. In the case of plasters, it is preferably from about 1 cm$^2$ to about 140 cm$^2$, particularly preferably from about 1.5 cm$^2$ to about 40 cm$^2$. In the case of cataplasms, it is preferably from about 140 cm$^2$ to about 280 cm$^2$. Additionally, although their shape may be in any form, it is preferably a square, a rectangle, a circle, an ellipse or the like.

Imidafenacin, which is the active ingredient of the present invention may be blended as either a soluble type or a mixture type of a soluble type and non-soluble type. It is not particularly limited as long as it has safety and can keep the safety, and can effect skin permeation of the active ingredient efficiently and continuously. Specifically, a soluble type imidafenacin alone is substantially used as the active ingredient, or a mixture type imidafenacin of its soluble type and non-soluble type may be blended as the active ingredient.

In general, although a non-soluble type agent is not related to the percutaneous absorption, the amount of an agent, which is quickly absorbed through the skin also becomes large as the content of a soluble type agent in the adhesive plaster becomes large. Therefore, it becomes possible to effect continuation of the effect of agent for a long time. In other words, duration of a pharmacological activity is restricted by the saturation solubility of an agent in the backbone for transdermal system. However, in the case of a backbone for transdermal system having low agent solubility, duration of the agent and continuation of stable effective blood concentration are not sufficient in some cases. In order to obtain proper agent persistency, its dose is increased by means in which an adhesive plaster having further high solubility is used; the drug-dissolved adhesive plaster is thickened; the agent content is increased; or area of the adhesive plaster contacting with the skin surface is enlarged. However, these methods have many problems in terms of the sense of incongruity, adhesiveness, skin irritation, economy and the like.

On the other hand, when the amount of an agent dissolved in the adhesive plaster is large and its percutaneous absorption rate is quick, a double layered adhesive plaster layer in which an adhesive plaster layer is further coated with a silicone resin or an polyacrylate or the like is used in some cases in order to obtain its persistency by delaying the absorption rate.

Concentration of a soluble type agent in the adhesive plaster directly exerts influence on the percutaneous absorption rate and is reduced by its absorption into the skin. Since an excess agent exceeding its saturation solubility in the adhesive plaster to be used is dispersed in the adhesive plaster as a non-soluble type agent, amount of the soluble type agent contained in the adhesive plaster is determined by the base to be used for external preparations.

On the other hand, a non-soluble type agent has a function to supply the soluble type agent, which is reduced by the skin absorption of the agent dissolved in the adhesive plaster into the adhesive plaster to compensate for the same. As a result, a high percutaneous absorption rate is maintained for a long time and the effective blood concentration is maintained for a long time.

Thus, according to the present invention, an adhesive single layer type percutaneous absorption type pharmaceutical preparation having percutaneous absorption property, stable pharmacokinetics pattern in blood and excellent agent effect persistence can be provided by selecting an adhesive plaster having high saturation solubility of the agent to prepare a pharmaceutical preparation which comprises substantially soluble type imidafenacin alone as the active ingredient, or when the absorption rate is quick, by preparing a double layered pharmaceutical preparation coated with an adhesive which does not contain imidafenacin, or by a pharmaceutical preparation which comprises a mixture type imidafenacin of a soluble type and a non-soluble type as the active ingredient.

According to the present invention, the soluble type imidafenacin means that imidafenacin is present under a completely dissolved state in the adhesive plaster, which specifically means that the adhesive plaster is uniform since crystals of imidafenacin are not observed in the adhesive plaster by visual observation or under a light microscope. Additionally, it is preferable that the agent can be kept under a completely dissolved state in the adhesive plaster without causing precipitation of the agent even at a high concentration. In order to keep a high concentration of imidafenacin by the soluble type, an additive agent may be further used. The additive agent is not limited as long as it is excellent in compatibility with the adhesive; can sufficiently dissolve imidafenacin; and does not cause periodical separation of the adhesive and additive agent. By this, This can maintain effective blood concentration for a long time due to superior percutaneous absorption rate in the initial stage of administration.

According to the present invention, the mixture type imidafenacin of soluble type and non-soluble type means that imidafenacin is present in the adhesive plaster as a mixture of a dissolved state and a non-dissolved state, which specifically a dissolved state and a crystalline state or a non-crystalline state. In order to compensate for the reduction of imidafenacin in the adhesive plaster by quick absorption of the soluble type, re-dissolution of the non-soluble type imidafenacin quickly occurs and the agent effect persistency is kept by the absorption of the dissolved imidafenacin. Ratio of the disappearing rate of the non-soluble type imidafenacin to the disappearing rate of total imidafenacin in the adhesive plaster is preferably about 0.1 or more. When the ratio of disappearing rates is small, since re-dissolution of the non-soluble type agent based on the reduction of the dissolution type agent becomes insufficient, persistency of the agent effect is not good.

Among the percutaneous absorption type pharmaceutical preparations of the present invention, the Plaster and Pressure Sensitive Adhesives can be produced by a general production method. For example, it can be produced by (1) a solvent method, (2) a heating method (hot melt method) or (3) a calender method. The solvent method is a method in which the active ingredient and the backbone for transdermal system are dissolved in hexane, rubber gasoline, ethyl acetate, alcohols, xylene, chloroform, water or the like solvent and spread to dry the solvent. Preferably, it is dried at from about 20°C to about 60°C for about 30 seconds to about 60 minutes. The heating method is a method in which the active ingredient and the backbone for transdermal system are dissolved and mixed at a high temperature, followed by spreading and cooling. The calender method is a method in which the active ingredient and the backbone for transdermal system are mixed with a mixer and spread with a calender roller. Additionally, when the viscosity is relatively low, the active ingredient and the backbone for transdermal system are mixed by a general mixer and are spread. Production methods of the pharmaceutical preparations of the present invention are not limited to the above methods. It may be produced by other efficient methods.

Since the percutaneous absorption type pharmaceutical preparations of the present invention have selective antagonism for muscarine receptors M3 and M1 in smooth muscles of the bladder, the trachea, digestive tracts and the like, they are useful for the prevention and/or treatment of not only pollakiurea and urinary incontinence accompanied by over active bladder (OAB) but also asthma, chronic obstructive pulmonary disease (COPD), irritable bowel syndrome (IBS) and the like. Accordingly, the percutaneous absorption type pharmaceutical preparations of the present invention have advantages in that the side effects which are generated by temporarily high agent (blood) concentration in the case of oral preparations and injections (e.g., dry mouth, feeling of residual urine, difficulty of urination, constipation, abnormal feeling in the eye, urinary retention and the like) can be avoided; their administration frequency is less than the oral preparations since they can be continuously absorbed from the skin; and that their application can be immediately stopped when a side effect is generated. Thus, the percutaneous absorption type pharmaceutical preparations of the present invention are greatly useful as the persistent preventive and/or therapeutic agents for pollakiurea and urinary incontinence accompanied by over active bladder (OAB), and asthma, COPD, IBS and the like, which improve qualities of life of old people and patients having a difficulty in oral administration. Particularly, with regard to prevention and/or treatment of COPD, since the percutaneous absorption type pharmaceutical preparations of the present invention enables effective continuous delivery of the agent from the blood vessel side to the alveolar peripheral tissues in comparison with inhalations, they become pharmaceutical preparations which are useful even for a patient having a difficulty in administering inhalations into peripheries by reduced respiratory function.

Examples

Although the following describes the present invention further in detail with Examples, the scope of the present invention is not restricted by these examples.

Example 1

An adhesive plaster was prepared by dispersing imidafenacin (20 mg), oleyl alcohol (200 mg) and Crotamiton (200 mg) in a silicone rubber (Q7-4501, manufactured by Dow Corning) (1.975 g). The adhesive plaster was spread on a release liner (Scotch Pack 9742, manufactured by 3M Health Care) to a certain thickness (about 125 $\mu$m) using an applicator. The adhesive plaster surface was dried for 20 minutes with hot air (about 50 to 60°C). By covering the thus dried adhesive plaster surface with the release liner and cutting it into a circular shape of 15 mm in diameter (1.766 cm$^2$), a pharmaceutical preparation (the main component content: 1 mg/10 cm$^2$) was prepared.

Example 2

An adhesive plaster was prepared by dispersing imidafenacin (20 mg), oleic aid (200 mg) and Crotamiton (200 mg) in the silicone rubber (Q7-4501, manufactured by Dow Corning) (1.975 g). The adhesive plaster was spread on a release liner (Scotch Pack9742, manufactured by 3M Health Care) to a certain thickness (about 125 $\mu$m) using an applicator. The adhesive plaster surface was dried for 20 minutes with hot air (about 50 to 60°C). By covering the thus dried adhesive plaster surface with the release liner and cutting it into a circular shape of 15 mm in diameter (1.766 cm$^2$), a pharmaceutical preparation (the main component content: 1 mg/10 cm$^2$) was prepared.

Examples 3(1) to 3(13)

Pharmaceutical preparations (the main component content: 1 mg/10 cm$^2$) of a circular shape of 15 mm in diameter (1.766 cm$^2$) were prepared by carrying out the same operation with Example 1 using imidafenacin (20 mg), adhesives shown in the following Table 1 and other bases for external preparations (however, when SIS and an ultra hypochromic rosin ester (ester gum) were used, chloroform (7.5 g) was used as the organic solvent).

Table 1

| Examples | Bases for external preparations | |
|---|---|---|
| | Adhesives | Others |
| 3(1) | SIS (790 mg)<br>Ultra hypochromic rosin ester (990 mg) | Diisopropyl adipate (200 mg) |
| 3(2) | SIS (790 mg)<br>Ultra hypochromic rosin ester (990 mg) | Diethyl sebacate (200 mg) |
| 3(3) | SIS (790 mg)<br>Ultra hypochromic rosin ester (990 mg) | Liquid paraffin (200 mg) |
| 3(4) | SIS (790 mg)<br>Ultra hypochromic rosin ester (990 mg) | Oleic acid (200 mg) |
| 3(5) | SIS (690 mg)<br>Ultra hypochromic rosin ester (890 mg) | Isopropyl myristate (200 mg)<br>Crotamiton (200 mg) |
| 3(6) | SIS (690 mg)<br>Ultra hypochromic rosin ester (890 mg) | Oleyl alcohol (200 mg)<br>Crotamiton (200 mg) |
| 3(7) | SIS (690 mg)<br>Ultra hypochromic rosin ester (890 mg) | Oleic acid (200 mg)<br>Crotamiton (200 mg) |
| 3(8) | SIL (2.225 g) | Isopropyl myristate (200 mg) |
| 3(9) | SIL (2.225 g) | Diethyl sebacate (200 mg) |
| 3(10) | SIL (2.225 g) | Oleyl alcohol (200 mg) |
| 3(11) | SIL (2.225 g) | Oleic acid (200 mg) |

(continued)

| Examples | Bases for external preparations | |
| --- | --- | --- |
| | Adhesives | Others |
| 3(12) | SIL (2.475 g) | - |
| 3(13) | NK (2.96 g) | Oleyl alcohol (200 mg) |

SIS: a styrene-isoprene-styrene block copolymer (SIS-5002, manufactured by Japan Synthetic Rubber Co., Ltd.)
Ultra hypochromic rosin ester (KE-311, manufactured by Arakawa Chemical Industries)
SIL: a silicone rubber (Q7-4501, manufactured by Dow Corning)
NK: Nikazol (an acryl system emulsion type adhesive, TS-620, manufactured by Nippon Carbide Industries Co., Inc.)

Examples 4(1) to 4(11)

Adhesive plasters were prepared by dispersing imidafenacin and the other bases for external preparations shown in the following Table 2 in SIL, or by dispersing imidafenacin, SIS, an ultra hypochromic rosin ester (ester gum) and the other bases for external preparations shown in the following Table 2 in chloroform (9.375 g). The adhesive plaster was spread on a release liner (Scotch Pack 9742, manufactured by 3M Health Care) to a certain thickness (about 125 $\mu$m) using an applicator. The adhesive plaster surface was dried for 20 minutes with hot air (about 50 to 60°C). By covering the thus dried adhesive plaster surface with the release liner and cutting it into a square shape of 2 cm$\times$2 cm (4 cm$^2$), each pharmaceutical preparation (the main component content: 0.5 mg/10 cm$^2$) was prepared.

Table 2

| Examples | Imidafenacin | Bases for external preparations | |
| --- | --- | --- | --- |
| | | Adhesives | Others |
| 4(1) | 25 mg | SIS (0.8625g)<br>Ultra hypochromic rosin ester (1.1125 g) | Oleic acid (125 mg)<br>Crotamiton (125 mg) |
| 4(2) | 25 mg | SIS (0.8625 g)<br>Ultra hypochromic rosin ester (1.1125 g) | Oleyl alcohol (125 mg)<br>Crotamiton (125 mg) |
| 4(3) | 50 mg | SIL (5.3125g) | Oleic acid (250 mg) |
| 4(4) | 50 mg | SIL (5.0000 g) | Oleic acid (250 mg)<br>Crotamiton (100 mg) |
| 4(5) | 50 mg | SIL (5.0000 g) | Oleic acid (250 mg)<br>Crotamiton (250 mg) |
| 4(6) | 50 mg | SIL (5.3125 g) | Oleyl alcohol (250 mg) |
| 4(7) | 50 mg | SIL (5.0000 g) | Oleyl alcohol (250 mg)<br>Crotamiton (100 mg) |
| 4(8) | 50 mg | SIL (5.0000 g) | Oleyl alcohol (250 mg)<br>Crotamiton (250 mg) |
| 4(9) | 25 mg | SIS (0.9875 g)<br>Ultra hypochromic rosin ester (1.2375 g) | Cetyl lactate (125 mg) |
| 4(10) | 25 mg | SIS (0.9875 g)<br>Ultra hypochromic rosin ester (1.2375 g) | Triacetin (125 mg) |
| 4(11) | 50 mg | SIL (6.1875 g) | - |

Example 5(1):

An adhesive plaster was prepared by mixing and dissolving a high molecular weight polyisobutylene (Vistanex MML-100, manufactured by Exxon Chemical Company) (3 g), a low molecular weight polyisobutylene (Oppanol B12SPN, manufactured by BASF Japan) (7 g), a styrene-isoprene-styrene block copolymer (SIS 5229, manufactured by Japan Synthetic Rubber) (20 g), an ester gum (KE-311, manufactured by Arakawa Chemical Industries) (10 g), an alicyclic saturated hydrocarbon resin (Alcon P-100, manufactured by Arakawa Chemical Industries) (20 g), liquid paraffin (Crystol 352, manufactured by Kaneda) (34 g), isopropyl myristate (manufactured by Nippon Oil & Fats) (5 g) and imidafenacin (1 g) in hexane. The adhesive plaster was spread on a silicone-treated liner (PET 75 $\mu$m) such that its thickness after drying became about 100 $\mu$m, and dried for 5 minutes with a hot air of about 50°C to evaporate hexane. By covering the thus dried adhesive plaster surface with a support (polyurethane 50 $\mu$m) and cutting it into a square shape of 3.2 x 3.2 mm (10 cm$^2$), a pharmaceutical preparation (the main component content: 1 mg/10 cm$^2$) was prepared.

Example 5(2)

An adhesive plaster was prepared by mixing and dissolving a high molecular weight polyisobutylene (Vistanex MML-100, manufactured by Exxon Chemical Company) (3 g), a low molecular weight polyisobutylene (Oppanol B12SPN, manufactured by BASF Japan) (7 g), a styrene-isoprene-styrene block copolymer (SIS 5229, manufactured by Japan Synthetic Rubber Ltd.) (20 g), an ester gum (KE-311, manufactured by Arakawa Chemical Industries Ltd.) (10 g), an alicyclic saturated hydrocarbon resin (Alcon P-100, manufactured by Arakawa Chemical Industries Ltd.) (20 g), liquid paraffin (Crystol 352, manufactured by Kaneda Corporation) (34 g), oleyl alcohol (manufactured by Nippon Oil & Fats) (2.5 g), Crotamiton (manufactured by Kongo Chemical) (2.5 g) and imidafenacin (1 g) in hexane. The adhesive plaster was spread on a silicone-treated liner (PET 50 $\mu$m) to be about 100 $\mu$m in thickness after drying, and dried for 5 minutes with a hot air of about 50°C to evaporate hexane. By covering the dried adhesive plaster surface with a support (PET 15 $\mu$m) and cutting it into a square shape of 3.2$\times$3.2 mm (10 cm$^2$), a pharmaceutical preparation (the main component content: 1 mg/10 cm$^2$) was prepared.

Example 5(3)

An adhesive plaster was prepared by mixing and dissolving a high molecular weight polyisobutylene (Vistanex MML-100, manufactured by Exxon Chemical Company) (3 g), a low molecular weight polyisobutylene (Oppanol B 12SPN, manufactured by BASF Japan) (7 g), a styrene-isoprene-styrene block copolymer (SIS 5229, manufactured by Japan Synthetic Rubber Co., Ltd.) (20 g), an ester gum (KE-311, manufactured by Arakawa Chemical Industries Ltd.) (10 g), an alicyclic saturated hydrocarbon resin (Alcon P-100, manufactured by Arakawa Chemical Industries) (20 g), liquid paraffin (Crystol 352, manufactured by Kaneda Corporation) (29 g), oleic acid (manufactured by NOF Corporation) (5 g), Crotamiton (manufactured by Kongo Chemical Co., Ltd.) (5 g) and imidafenacin (1 g) in hexane. The adhesive plaster was spread on a silicone-treated liner (PET 75 $\mu$m) to be about 100 $\mu$m in thickness after drying, and dried for 5 minutes with a hot air of about 50°C to evaporate hexane. By covering the thus dried adhesive plaster surface with a support (PET 4 $\mu$m/PE 20 $\mu$m) and cutting it into a square shape of 3.2$\times$3.2 mm (10 cm$^2$), a pharmaceutical preparation (the main component content: 1 mg/10 cm$^2$) was prepared.

Example 5(4)

An adhesive plaster was prepared by mixing and dissolving a silicone rubber (Q7-4501, manufactured by Dow Corning) (74 g), an ester gum (KE-311, manufactured by Arakawa Chemical Industries Ltd.) (20 g), isopropyl myristate (manufactured by Nippon Oil & Fats) (2.5 g), Crotamiton (Kongo Chemical Co., Ltd.)(2.5 g) and imidafenacin (1 g). The adhesive plaster was spread on a silicone-treated liner (PE 80 $\mu$m) to be about 100 $\mu$m in thickness after drying, and dried for 5 minutes with a hot air of about 50°C to evaporate the solvent. By covering the thus dried adhesive plaster surface with a support (PET 4 $\mu$m/40 g woven fabric) and cutting it into a square shape of 3.2$\times$3.2 mm (10 cm$^2$), a pharmaceutical preparation (the main component content: 1 mg/10 cm$^2$) was prepared.

Example 5(5)

An adhesive plaster was prepared by mixing and dissolving a silicone rubber (Q7-4501, manufactured by Dow Corning) (74 g), an ester gum (KE-311, manufactured by Arakawa Chemical Industries Ltd.) (10 g), an alicyclic saturated hydrocarbon resin (Alcon P-100, manufactured by Arakawa Chemical Industries Ltd.) (10 g), oleyl alcohol (manufactured by Nippon Oil & Fats) (2.5 g), Crotamiton (Kongo Chemical) (2.5 g) and imidafenacin (1 g). The adhesive plaster was spread on a silicone-treated liner (fine quality paper 100 $\mu$m) such that its thickness to be about 100 $\mu$m in thickness after drying, and dried for 5 minutes with a hot air of about 50°C to evaporate the solvent. By covering the thus dried adhesive plaster surface with a support (PET 5 $\mu$m/20 g non-woven fabric) and cutting it into a square shape of 3.2$\times$3.2 mm (10 cm$^2$), a pharmaceutical preparation (the main component content: 1 mg/10 cm$^2$) was prepared.

Examples 5(6) to 5(13)

Each pharmaceutical preparations (the main component content: 1 mg/10 cm$^2$), each having a square shape of 3.2 x 3.2 mm (10 cm$^2$), were prepared by carrying out the same operations with Examples 5(1) to 5(5) using imidafenacin (1 g) and the bases for external preparations shown in Table 3.

Table 3

| Examples | Bases for external preparations | |
|---|---|---|
| | Adhesives | Others |
| 5(6) | High molecular weight polyisobutylene (3g) Low molecular weight polyisobutylene (7g) SIS (20 g) Rosin ester (10 g) Alicyclic saturated hydrocarbon resin (20 g) | Liquid paraffin (34 g) Oleyl alcohol (5 g) |
| 5(7) | High molecular weight polyisobutylene (3g) Low molecular weight polyisobutylene (7g) SIS (20 g) Rosin ester (10 g) Alicyclic saturated hydrocarbon resin (20 g) | Liquid paraffin (34 g) Oleic acid (5 g) |
| 5(8) | High molecular weight polyisobutylene (3g) Low molecular weight polyisobutylene (7g) SIS (20 g) Rosin ester (10 g) Alicyclic saturated hydrocarbon resin (20 g) | Liquid paraffin (34 g) Crotamiton (5g) |
| 5(9) | High molecular weight polyisobutylene (3g) Low molecular weight polyisobutylene (7g) SIS (20 g) Rosin ester (10 g) Alicyclic saturated hydrocarbon resin (20 g) | Liquid paraffin (34 g) Oleic acid (2.5 g) Crotamiton (2.5 g) |
| 5(10) | High molecular weight polyisobutylene (3g) Low molecular weight polyisobutylene (7g) SIS (20 g) Rosin ester (10 g) Alicyclic saturated hydrocarbon resin (20 g) | Liquid paraffin (34 g) Cetyl lactate (5g) |
| 5(11) | High molecular weight polyisobutylene (3g) Low molecular weight polyisobutylene (7g) SIS (20 g) Rosin ester (10 g) Alicyclic saturated hydrocarbon resin (20 g) | Liquid paraffin (34 g) Cetyl lactate (2.5 g) Crotamiton (2.5 g) |
| 5(12) | High molecular weight polyisobutylene (3g) Low molecular weight polyisobutylene (7g) SIS (20 g) Rosin ester (10 g) Alicyclic saturated hydrocarbon resin (20 g) | Liquid paraffin (34 g) Triacetin (5 g) |
| 5(13) | High molecular weight polyisobutylene (3g) Low molecular weight polyisobutylene (7g) SIS (20 g) Rosin ester (10 g) Alicyclic saturated hydrocarbon resin (20 g) | Liquid paraffin (34 g) Triacetin (2.5 g) Crotamiton (2.5 g) |

Example 6(1)

A high molecular weight polyisobutylene (Vistanex MML-100, manufactured by Exxon Chemical Company) (3 g), a low molecular weight polyisobutylene (Oppanol BT2SPN, manufactured by BASF Japan) (6 g), a styrene-isoprene-styrene block copolymer (SIS 5229, manufactured by Japan Synthetic Rubber Co., Ltd.) (20 g), an ester gum (KE-311, manufactured by Arakawa Chemical Industries Ltd.) (7 g), an alicyclic saturated hydrocarbon resin (Alcon P-100, manufactured by Arakawa Chemical Industries Ltd.) (21 g) and liquid paraffin (Crystol 352, manufactured by Kaneda Corporation) (37 g) were mixed with hexane having almost the same weight with these base components and dissolved therein with stirring (solution A). Imidafenacin (1 g) and isopropyl myristate (5 g) were mixed and stirred at 90°C for about 10 minutes (solution B). The solution B was cooled down to 30°C and then added to the solution A to prepare an adhesive plaster. In order to adjust the plaster weight to be 100 g/m$^2$, the adhesive plaster was spread using an automatic coating device (manufactured by Tester Sangyo Co., Ltd.) on a silicone-treated liner (PET 75 $\mu$m, manufactured by Toray Film Processing) to be about 100 $\mu$m in thickness after drying, and spontaneously dried. By covering the dried adhesive plaster surface with a support (PET/non-woven fabric) and cutting it into a square shape of 3.2×3.2 mm (10 cm$^2$), a pharmaceutical preparation (the main component content: 1 mg/10 cm$^2$) was prepared.

Examples 6(2) to 6(5)

Pharmaceutical preparations (the main component content: 1 mg/10 cm$^2$) were prepared by carrying out the same operation with Example 6(1) using the components shown in Table 4 instead of isopropyl myristate.

Table 4

| Examples | Components |
|---|---|
| 6(2) | Oleyl alcohol (5 g) |
| 6(3) | Purified oleic acid (manufactured by NOF Corporation, 5 g) |
| 6(4) | Crotamiton (5 g) |
| 6(5) | Ethyl lactate (5 g) |

Example 7(1)

A high molecular weight polyisobutylene (Vistanex MML-100, manufactured by Exxon Chemical Company) (3 g), a low molecular weight polyisobutylene (Oppanol B12SPN, manufactured by BASF Japan) (4 g), a styrene-isoprene-styrene block copolymer (SIS 5229, manufactured by Japan Synthetic Rubber Co., Ltd.) (20 g), an ester gum (KE-311, manufactured by Arakawa Chemical Industries Ltd.) (2 g), an alicyclic saturated hydrocarbon resin (Alcon P-100, manufactured by Arakawa Chemical Industries Ltd.) (26 g) and liquid paraffin (Crystol 352, manufactured by Kaneda Corporation) (38.5 g) were mixed with hexane having almost the same weight with these base components and dissolved therein with stirring (solution A). Imidafenacin (1 g), N-methyl-2-pyrrolidone (3 g) and propylene glycol (2.5 g) were mixed and stirred at 75°C for about 10 minutes (solution B). The solution B was cooled to 30°C and then added to the solution A to prepare an adhesive plaster. In order to adjust the plaster weight to be 100 g/m$^2$, the adhesive plaster was spread using an automatic coating device (manufactured by Tester Sangyo Co., Ltd.) on a silicone-treated liner (PET 75 $\mu$m, manufactured by Toray Advanced Film Co., Ltd.) to be about 100 $\mu$m in thickness after drying, and spontaneously dried. By covering the thus dried adhesive plaster surface with a support (PET/non-woven fabric) and cutting it into a square shape of 3.2×3.2 mm (10 cm$^2$), a pharmaceutical preparation (the main component content: 1 mg/10 cm$^2$) was prepared.

Examples 7(2) to 7(31)

Pharmaceutical preparations (the main component content: from 0.1 to 1 mg/10 cm$^2$) were prepared by carrying out the same operation with Example 7(1) using the solution A and solution B shown in Tables 5 and 6.

Table 5

| Examples | Solution A | | Solution B |
|---|---|---|---|
| | High molecular weight polyisobutylene (g)/ low molecular weight polyisobutylene (g)/ SIS (g)/ ester gum (g)/ alicyclic saturated hydrocarbon resin (g)/ liquid paraffin (g) | Additive agents (g) | Imidafenacin (g)/ N-methyl-2-pyrrolidone (g)/ propylene glycol (g) |
| 7(2) | 3/4/20/2/26/37.5 | Purified oleic acid (1) | 1/3/2.5 |
| 7(3) | 3/4/20/2/26/35.5 | Purified oleic acid (3) | 1/3/2.5 |
| 7(4) | 3/4/20/2/26/35.5 | Isopropyl myristate (3) | 1/3/2.5 |
| 7(5) | 3/4/20/2/26/35.5 | Oleyl alcohol (3) | 1/3/2.5 |
| 7(6) | 3/4/20/2/26/35.5 | Crotamiton (3) | 1/3/2.5 |
| 7(7) | 3/4/20/2/26/35.5 | Cetyl lactate (3) | 1/3/2.5 |
| 7(8) | 3/4/20/2/26/35.5 | Triacetin (3) | 1/3/2.5 |
| 7(9) | 3/4/20/2/26/36.4 | Purified oleic acid (3) | 0.1/3/2.5 |
| 7(10) | 3/4/20/2/26/36.3 | Purified oleic acid (3) | 02/3/25 |
| 7(11) | 3/4/20/2/26/36 | Purified oleic acid (3) | 0.5/3/2.5 |
| 7(12) | 3/4/20/2/26/35.3 | Purified oleic acid (3), BHT (0.2) | 1/312.5 |
| 7(13) | 3/4/20/3/26/37.8 | BHT (0.2) | 1/5/0 |
| 7(14) | 3/4/20/3/26/36.8 | Purified oleic acid (1), BHT (0.2) | 1/5/0 |
| 7(15) | 3/4/20/3/26/34.8 | Purified oleic acid (3), BHT (0.2) | 1/5/0 |
| 7(16) | 3/4/20/3/26/34.8 | BHT (0.2) | 1/8/0 |
| 7(17) | 3/4/20/3/26/33.8 | Purified oleic acid (1), BHT (0.2) | 1/8/0 |

Table 6

| Examples | Solution A | | Solution B |
|---|---|---|---|
| | High molecular weight polyisobutylene (g)/ low molecular weight polyisobutylene (g)/ SIS (g)/ ester gum (g)/ alicyclic saturated hydrocarbon resin (g)/ liquid paraffin (g) | Additive agents (g) | Imidafenacin (g)/ N-methyl-2-pyrrolidone (g)/ propylene glycol (g) |
| 7(18) | 3/4/20/3/26/3 1.8 | Purified oleic acid (3), BHT (0.2) | 1/8/0 |
| 7(19) | 3/4/20/3/26/39.8 | BHT (0.2) | 0.5/0/3.5 |
| 7(20) | 3/4/20/3/26/38.8 | Purified oleic acid (1), BHT (0.2) | 0.5/0/3.5 |
| 7(21) | 3/4/20/3/26/36.8 | Purified oleic acid (3), BHT (0.2) | 0.5/0/3.5 |
| 7(22) | 3/4/20/3/26/38.3 | BHT (0.2) | 0.5/5/0 |
| 7(23) | 3/4/20/3/26/37.3 | Purified oleic acid (1), BHT (0.2) | 0.5/5/0 |
| 7(24) | 3/4/20/3/26/35.3 | Purified oleic acid (3), BHT (0.2) | 0.5/5/0 |
| 7(25) | 3/4/20/3/26/40.8 | BHT (0.2) | 0.5/2.5/0 |

(continued)

| Examples | Solution A | | Solution B |
|---|---|---|---|
| | High molecular weight polyisobutylene (g)/ low molecular weight polyisobutylene (g)/ SIS (g)/ ester gum (g)/ alicyclic saturated hydrocarbon resin (g)/ liquid paraffin (g) | Additive agents (g) | Imidafenacin (g)/ N-methyl-2-pyrrolidone (g)/ propylene glycol (g) |
| 7(26) | 3/4/20/3/26/39.8 | Purified oleic acid (1), BHT (0.2) | 0.5/2.5/0 |
| 7(27) | 3/4/20/3/26/37.8 | Purified oleic acid (3), BHT (0.2) | 0.5/2.5/0 |
| 7(28) | 3/6/20/11/23/29.8 | Purified oleic acid (3), BHT (0.2) | 0.5/0/3.5 |
| 7(29) | 3/6/20/11/23/27.8 | Purified oleic acid (3), BHT (0.2) | 0.5/2/3.5 |
| 7(30) | 3/6/20/11/23/27.8 | Purified oleic acid (5), BHT (0.2) | 0.5/0/3.5 |
| 7(31) | 3/6/20/11/23/25.8 | Purified oleic acid (3), BHT (0.2) | 1/0/7 |

High molecular weight polyisobutylene: Vistanex MML-100, manufactured by Exxon Chemical Company
Low molecular weight polyisobutylene: Oppanol B12SPN, manufactured by BASF Japan
SIS: a styrene-isoprene-styrene block copolymer (SIS 5229, manufactured by Japan Synthetic Rubber Co., Ltd.)
Ester gum: KE-311, manufactured by Arakawa Chemical Industries Ltd. Alicyclic saturated hydrocarbon resin: Alcon P-100, manufactured by Arakawa Chemical Industries Ltd.
Liquid paraffin: Crystol 352, manufactured by Kaneda Corporation
Purified oleic acid: manufactured by Nippon Oil & Fats
BHT: dibutylhydroxytoluene

Examples 8(1) to 8(12)

Pharmaceutical preparations (the main component content: from 0.5 to 1 mg/10 cm$^2$) were prepared by carrying out the same operation with Example 7(1) using the solution A and solution B shown in Table 7.

Table 7

| Examples | Solution A | | Solution B |
|---|---|---|---|
| | High molecular weight polyisobutylene (g)/ low molecular weight polyisobutylene (g)/ SIS (g)/ ester gum (g)/ alicyclic saturated hydrocarbon resin (g) / liquid paraffin (g) | Additive agents (g) | Imidafenacin (g)/ N-methyl-2-pyrrolidone (g)/ propylene glycol (g) |
| 8(1) | 3/6/20/11/23/30 | Purified oleic acid (3) | 0.5/0/3.5 |
| 8(2) | 3/6/20/11/23/28 | Purified oleic acid (3) | 0.5/2/3.5 |
| 8(3) | 3/6/20/11/23/28 | Purified oleic acid (5) | 0.5/0/3.5 |
| 8(4) | 3/6/20/22/23/17 | Purified oleic acid (3) | 0.5/2/3.5 |
| 8(5) | 3/6/20/22/23/17 | Purified oleic acid (5) | 0.5/0/3.5 |
| 8(6) | 3/6/20/22/23/15 | Purified oleic acid (3) | 1/0/7 |
| 8(7) | 3/6/20/17/23/22 | Purified oleic acid (3) | 0.5/2/3.5 |
| 8(8) | 3/6/20/22/23/16.8 | Purified oleic acid (3), DL-$\alpha$-tocopherol (0.2) | 0.5/2/3.5 |
| 8(9) | 3/6/20/17/20/25 | Purified oleic acid (3) | 0.5/2/3.5 |

(continued)

| Examples | Solution A | | Solution B |
|---|---|---|---|
| | High molecular weight polyisobutylene (g)/ low molecular weight polyisobutylene (g)/ SIS (g)/ ester gum (g)/ alicyclic saturated hydrocarbon resin (g) / liquid paraffin (g) | Additive agents (g) | Imidafenacin (g)/ N-methyl-2-pyrrolidone (g)/ propylene glycol (g) |
| 8(10) | 3/6/20/17/20/24.8 | Purified oleic acid (3), BHT (0.2) | 0.5/2/3.5 |
| 8(11) | 3/6/20/19/19/23.8 | Purified oleic acid (3), BHT (0.2) | 0.5/2/3.5 |
| 8(12) | 3/6/20/17/20/24.8 | Purified oleic acid (3), DL-α-tocopherol (0.2) | 0.5/2/3.5 |

High molecular weight polyisobutylene: Vistanex MML-100, manufactured by Exxon Chemical Company
Low molecular weight polyisobutylene: Oppanol B12SPN, manufactured by BASF Japan
SIS: a styrene-isoprene-styrene block copolymer (SIS 5229, manufactured by Japan Synthetic Rubber Co., Ltd.)
Ester gum: KE-311, manufactured by Arakawa Chemical Industries Ltd.
Alicyclic saturated hydrocarbon resin: Alcon P-100, manufactured by Arakawa Chemical Industries Ltd.
Liquid paraffin: Crystol 352, manufactured by Kaneda Corporation
Purified oleic acid: manufactured by Nippon Oil & Fats
BHT: dibutylhydroxytoluene

Example 9(1)

An adhesive plaster was prepared by mixing and dissolving a silicone rubber (Q7-4501, manufactured by Dow Corning) (74 g), an ester gum (KE-311, manufactured by Arakawa Chemical Industries Ltd.) (10 g), an alicyclic saturated hydrocarbon resin (Alcon P-100, manufactured by Arakawa Chemical Industries Ltd.) (7 g), purified oleic acid (manufactured by Nippon Oil & Fats) (3 g), N-methyl-2-pyrrolidone (2 g), propylene glycol (3.5 g) and imidafenacin (0.5 g). The adhesive plaster was spread on a silicone-treated liner (fine quality paper 100 $\mu$m) to be about 100 $\mu$m in thickness after drying, and spontaneously dried to evaporate the solvent. By covering the dried adhesive plaster surface with a support (PET 5 $\mu$m/20 g non-woven fabric) and cutting it into a square shape of 3.2 x 3.2 mm (10 cm$^2$), a pharmaceutical preparation (the main component content: 0.5 mg/10 cm$^2$) was prepared.

Example 9(2) to Example 9(8)

Pharmaceutical preparations (the main component content: 0.5 mg/10 cm$^2$) were prepared by carrying out the same operation with Example 9(1) using the components shown in Table 8 instead of purified oleic acid. However, when DL-α-tocopherol was added, amount of the alicyclic saturated hydrocarbon resin was changed to 6.8 g.

Table 8

| Examples | Components |
|---|---|
| 9(2) | Purified oleic acid (manufactured by Nippon Oil & Fats, 3 g) DL-α-tocopherol (0.2 g) |
| 9(3) | Triacetin (3 g) |
| 9(4) | Triacetin (3 g) DL-α-tocopherol (0.2 g) |
| 9(5) | Cetyl lactate (3 g) |
| 9(6) | Cetyl lactate (3 g) DL-α-tocopherol (0.2 g) |
| 9(7) | Oleyl alcohol (3 g) |

(continued)

| Examples | Components |
|---|---|
| 9(8) | Oleyl alcohol (3 g) <br> DL-$\alpha$-tocopherol (0.2 g) |

Test Example 1: Percutaneous permeation test

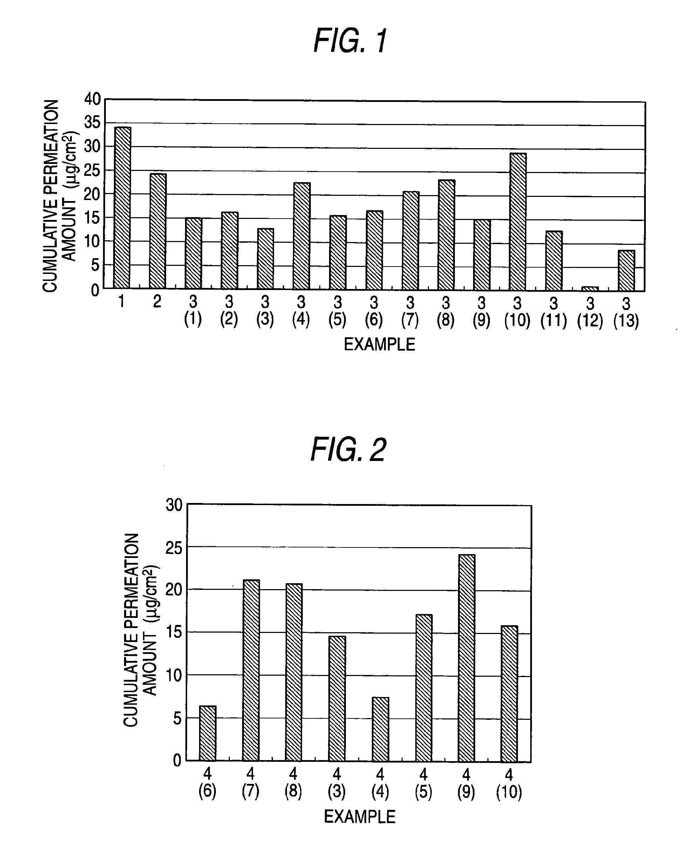

The hairless skin extracted from the abdominal region of each of male hairless rats (7 weeks of age, HWY strain, n = 4) was attached to a Franz type cell (1.5 cm in diameter). Each of the aforementioned pharmaceutical preparation prepared in Examples 1, 2, 3(1) to (13) and 4(6) to (10) was stuck to the keratin layer of the hairless skin extracted from the abdominal region. A 10 mM phosphate buffer (pH 6.0) was applied to the cell of dermal layer side. The phosphate buffer (100 $\mu$l) was periodically collected from the dermal layer side cell, and cumulative permeation amount of imidafenacin during every hour after 26 hours was determined to calculate average values, by an HPLC method under the following conditions. The results on the cumulative permeation amount after 26 hours are shown in Fig. 1 and Fig. 2.

Column: TSKgel ODS-80TM,
Column temperature: 50°C,
Mobile phase: 0.01 M phosphoric acid aqueous solution:acetonitrile = 73:27,
Detection wavelength: 220 nm
Injection quantity: 80$\mu$l
Retention time: 6.6 minutes

Based on the result, although high cumulative permeation amount was not obtained by the pharmaceutical preparation prepared in Example 3(12) in which the backbone for transdermal system consisted of SIL alone, and the pharmaceutical preparation prepared in Example 3(13) in which the backbone for transdermal system consisted of NK and oleyl alcohol, but high permeation was obtained by the pharmaceutical preparations in which the adhesive among other bases for external preparations was produced using SIS or SIL.

Additionally, as shown in the following Table 9, high cumulative permeation amount was obtained also by the pharmaceutical preparations produced in Examples 6(3), 7(1) to 7(4), 7(6), 7(9) to 7(12), 7(15), 7(18), 7(20) and 7(21), 7(24), and 7(27) to 7(31).

Table 9

| Examples | Cumulative permeation amount after 26 hours ($\mu$g/cm$^2$) |
|---|---|
| 6(3) | 31.3 $\pm$ 2.2 |
| 7(1) | 28.6 $\pm$ 1.9 |
| 7(2) | 34.7 $\pm$ 3.8 |
| 7(3) | 19.9 $\pm$ 2.6 |
| 7(4) | 15.6 $\pm$ 1.1 |
| 7(6) | 14.9 $\pm$ 4.9 |
| 7(9) | 8.0 $\pm$ 0.5 |
| 7(10) | 14.2 $\pm$ 0.5 |
| 7(11) | 24.3 $\pm$ 0.1 |
| 7(12) | 26.3 $\pm$ 2.2 |
| 7(15) | 29.8 $\pm$ 1.0 |
| 7(18) | 18.4 |
| 7(20) | 14.9 $\pm$ 1.9 |
| 7(21) | 29.8 $\pm$ 0. 6 |
| 7(24) | 22.9 $\pm$ 0.3 |

(continued)

| Examples | Cumulative permeation amount after 26 hours ($\mu$g/cm$^2$) |
|---|---|
| 7(27) | 18.2 $\pm$ 1.5 |
| 7(28) | 35.7 $\pm$ 3.4 |
| 7(29) | 41.8 $\pm$ 1.6 |
| 7(30) | 44.8 $\pm$ 3.4 |
| 7(31) | 50.1 $\pm$ 1,3 |

Test Example 2: Percutaneous permeation test

The skin of each of female minipigs (5 weeks of age, Yucatan micropig, n = 3) was cut into a size of about 5 cm square and soaked in a hot water of 60°C for 1 minute. Thereafter, the epidermis and the dermis were peeled off. The released epidermis was put on a Millipore filter (25 mm in diameter, 0.45 $\mu$l in pore size) which was soaked in 10 mM phosphate buffer (pH 6.0). The pharmaceutical preparation of the present invention was stuck to the epidermis and attached to a Franz type cell (1.5 cm in diameter). The phosphate buffer (100 $\mu$l) was periodically collected from the dermal layer side cell, and cumulative permeation of imidafenacin after 34 hours was determined by HPLC under the same conditions with Test Example 1.

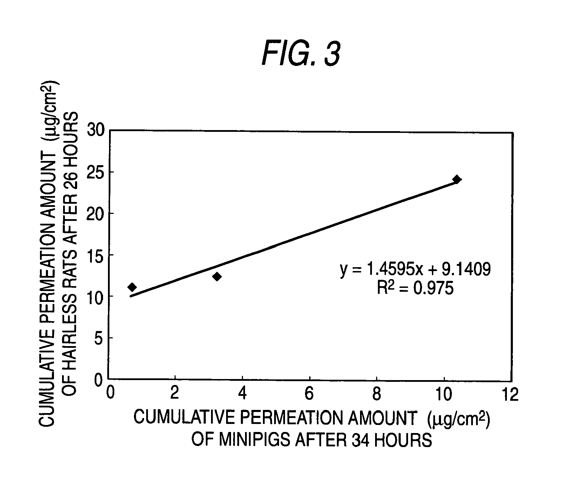

As a result, sufficient cumulative permeation amount was obtained by the pharmaceutical preparations of the present invention also in minipigs. For example, average value of the cumulative permeation of the pharmaceutical preparation of Example 2 was 10.27 $\mu$g/cm$^2$. Additionally, as shown in Fig. 3, good correlation was obtained between the cumulative permeation amount using minipig percutaneous absorption and the cumulative permeation amount using hairless rat percutaneous absorption of the pharmaceutical preparations produced in Reference Example 1, which is described later, Example 3(11) and Example 2.

Reference Example 1:

An adhesive plaster was prepared by dispersing imidafenacin (25 mg), a styrene-isoprene-styrene block copolymer (0.9875 g, SIS-5002, manufactured by Japan Synthetic Rubber Co., Ltd.), an ultra hypochromic rosin ester (ester gum) (1.2375 g, KE311, manufactured by Arakawa Chemical Industries Corporation) and isopropyl myristate (125 mg) in chloroform (9.375 g). The adhesive plaster was spread on a release liner (Scotch Pack 9742, manufactured by 3M Health Care) to a thickness of about 125 $\mu$m using an applicator. The adhesive plaster surface was dried for 20 minutes with hot air (about 50 to 60°C). By covering the dried adhesive plaster surface with the release liner and cutting it into a circular shape of 15 mm in diameter (1.766 cm$^2$), a pharmaceutical preparation (the main component content: 1 mg/10 cm$^2$) was prepared.

Test Example 3: Blood concentration

The abdominal region skin of each of male hairless rats (10 weeks of age, HWY/S1c, n = 6) was shaved using an electric hair clipper. Each of the pharmaceutical preparations produced in the aforementioned Examples was stuck to the abdominal part and then blocked up for 48 hours by wrapping with a non-woven fabric adhesive bandage (Mesh Pour, manufactured by Nichiban Co., Ltd.). The pharmaceutical preparation was removed after 48 hours. After 3, 6, 9, 24, 32 or 48 hours of the sticking, blood (about 0.3 ml) was collected from jugular vein without anesthesia using a heparin-treated polypropylene disposable syringe, and the blood plasma was transferred to a polypropylene container. The thus obtained plasma was immediately ice-cooled and subjected to cryopreservation. Thereafter, blood concentration of imidafenacin (ng/ml) at each time was measured, and its mean value from 6 samples was calculated.

As a result, although Example 4(11) in which the backbone for transdermal system is the adhesive alone was hardly absorbed and the object was not attained, it was confirmed that the pharmaceutical preparations of the present invention other than it were continuously absorbed into blood during the 48 hours with sticking and a concentration of a certain level or more was maintained. For example, in the case of the pharmaceutical preparation produced in Example 4(3), its blood concentration after 48 hours of the sticking showed a persistent blood concentration within a range of from 0.152 $\pm$ 0.049 ng/ml to 0.766 $\pm$ 0.594 ng/ml.

Additionally, the pharmaceutical preparations produced in Examples 7(15), 7(21), 7(24) and 7(27) to 7(31) showed a persistent blood concentration within the range shown in Table 10. In this connection, with regard to the pharmaceutical preparations produced in Examples 7(15), 7(21), 7(24) and 7(27), each of the pharmaceutical preparations obtained by the aforementioned production methods was cut into a quadrangle of 2 x 2 mm (4 cm$^2$) and used in the evaluation.

With regard to Examples 7(28) to 7(31), each of the 10 cm$^2$ pharmaceutical preparations was directly stuck and evaluated.

Table 10

| Examples | Blood concentration (ng/ml) |
|---|---|
| 7(15) | $0.052 \pm 0.056$ to $0.481 \pm 0.280$ |
| 7(21) | $0.092 \pm 0.061$ to $1.225 \pm 1.060$ |
| 7(24) | $0.182 \pm 0.165$ to $0.635 \pm 0.408$ |
| 7(27) | $0.037 \pm 0.033$ to $0.538 \pm 0.351$ |
| 7(28) | $0.469 \pm 0{,}275$ to $3.081 \pm 1.578$ |
| 7(29) | $0.401 \pm 0.349$ to $4.713 \pm 3.919$ |
| 7(30) | $0.577 \pm 0.295$ to $5.273 \pm 3.772$ |
| 7(31) | $0.913 \pm 0.464$ to $5.912 \pm 3.058$ |

Test Example 4: Evaluation of hairless rat skin primary irritation

In the Test Example 3, each of the pharmaceutical preparation produced in the aforementioned Examples was stuck to the abdominal region of each male hairless rat for 48 hours, and then the skin reactions at the time of the removal of the pharmaceutical preparation (0 hour), 24 hours after its removal, 48 hours after its removal and 72 hours after its removal were observed to calculate P.I.I. and evaluated in accordance with the Draize's evaluation standrd [cf. J. Pharmacal. Exp. Ther., 83, 377 - 390, (1944)] shown in Table 11.

Table 11

| A. | Erythema and crust | Score |
|---|---|---|
| | No erythema | 0 |
| | Very slight erythema (barely recognizable) | 1 |
| | Distinct erythema | 2 |
| | Middle to strong erythema | 3 |
| | Dark red strong erythema and slight crust symptom (injury in deep region) | 4 |
| B. | Edema | |
| | No edema | 0 |
| | Very slight edema (barely recognizable) | 1 |
| | Distinct edema (which can be distinguished from periphery) | 2 |
| | Middle to strong edema (swells about 1 mm) | 3 |
| | Strong edema (swells of 1 mm or more, also widens toward periphery) | 4 |

Mean score of each analyte was calculated. Furthermore, average score of all analytes was calculated from the average score of each analyte and used as the primary irritation index (P.I.I.).

$$\text{Average score of each analyte} = \text{total of the score of each analyte}/4$$

P.I.I. = total of the average score of each analyte/6

Safety sections were set to (i) no irritation when P.I.I, is 0, (ii) weak irritant when it is exceeding 0 and 2 or less, (iii) medium irritant when it is exceeding 2 and 5 or less and (iv) strong irritant when it exceeds 5. When the safety section is 0 or exceeding 0 and 2 or less, it can be judged that there is no problem on the irritation.

As a result, the skin primary irritation index of all of the pharmaceutical preparations of the present invention was 1 or less. It shows weak irritation and has no problems.

Test Example 5: Evaluation of rabbit skin primary irritation

Male rabbits (10 weeks of age, from 2.00 to 3.50 kg in body weight) were used. The sticking regions were prepared at 6 positions, by removing the dorsal region hair using electric hair clippers on the day before the sticking (before 24 hours). Three positions among them were used as normal skins, and the remaining three positions were used as injured skins by scratching the keratin layer with a needle. Each the pharmaceutical preparations produced in Examples 4(2), 4(3) and 4(4) and negative control pharmaceutical preparations was stuck to the normal skins and injured skins and then fixed using the Nichiban adhesive bandage. The sticking time was set to 24 hours. After removal of the pharmaceutical preparations, the remaining agent and the like were lightly wiped using absorbent cotton wetted with slightly hot water. The skin reactions after 30 minutes of the removal, 24 hours after the removal, 48 hours after the removal and 72 hours after the removal were observed to calculate P.I.I. and evaluated in accordance with the aforementioned Draize's evaluation standard. As the negative control pharmaceutical preparations, 3 kinds of pharmaceutical preparations produced by the same methods with the pharmaceutical preparations of Examples 4(2), 4(3) and 4(4) except that the active ingredient was not contained were used.
As a result, the skin primary irritation index of all of the pharmaceutical preparations of the present invention was 1 or less. It shows weak irritation and has no problems.
Additionally, when the same test was carried out, for example, by sticking the pharmaceutical preparations of Examples 7(29) and 7(30) and respective negative control pharmaceutical preparations to the normal skins and injured skins for 48 hours, the skin primary irritation index of each case was 1 or less. It shows weak irritation and has no problems.

Test Example 6: Adhesive strength test

Measurement was carried out by using an Instron type tensile tester (AUTOGRAPH AGS-IKND; manufactured by Shimadzu Corporation). A tip of the longer side of an auxiliary paper (25 mm in width, 100 mm in length) was stuck to an area of 10 mm from the side of the pharmaceutical preparation of the present invention (20 mm × 20 mm). The remaining adhering face of the pharmaceutical preparation was stuck to the center of a test plate made of a phenol resin (25 mm in width, 125 mm in length, 5 mm in thickness), which had been allowed to stand at ordinary temperature for 30 minutes in advance, in such a manner that respective longer sides of the test plate and auxiliary paper became the same direction. A rubber roller (850 g) was immediately allowed to pass over the pharmaceutical preparation twice at a rate of 300 mm per minute. After allowing it to stand at ordinary temperature for 30 minutes, loads of 4 times were measured at intervals of 1 mm by continuously peeling at a rate of 100 mm per minute with the tensile tester wherein free end of the auxiliary paper stuck to the test plate was folded to an angle of 180°, and free end of the pharmaceutical preparation was tightly held to the upper side, while the test plate was tightly held at the lower side by buckles. For example, the average value was 240 g in the case of the pharmaceutical preparation of Example 4(2).

Test Example 7: Measurement of effective blood concentration in guinea pigs

<Intravesical pressure measuring method>

Male Std:Hartley guinea pigs (Japan SLC Inc., week of age at the time of use: 6 weeks of age, from 340.6 to 488.0 g in body weight) were anesthetized with urethane. The abdominal region was subjected to median incision, and then both sides of ureters were ligated in order to prevent inflow of urine from the kidney to the bladder. After ligation of the bladder cervix, a catheter for intravesical pressure measurement use was inserted from the bladder apex and fixed by ligation. Thereafter, in order to measure intravesical pressure, 1 ml of physiological saline was injected into the bladder. Next, a catheter was indwelled in the left jugular vein. The intravesical pressure was measured via an amplifier for strain pressure use (Nihon Kohden Corporation, CARRIER ANPLIFIER AP-601G) by connecting the catheter for intravesical pressure measuring use to a pressure transducer (Nihon Kohden Corporation, LIFE KIT DX-100). By inserting a catheter into the jugular vein, imidafenacin was administered by continuous intravenous infusion (100 $\mu$l/kg/min). After 75 minutes of the commencement of administration of the agent liquid, methacholine (10 $\mu$g/kg) was intravenously administered to induce bladder contraction. The bladder contraction inhibitory activity was evaluated using peak height of the bladder contraction induced by methacholine as the index.
As a result, imidafenacin showed a dose-dependent inhibitory activity at 0.03, 0.1 and 0.3 $\mu$g/kg/min, and the ID$_{50}$ value was 0.064 $\mu$g/kg/min. Additionally, the blood concentration at around the ID$_{50}$ value was about 1 ng/ml.
In this connection, the blood concentration measurement was carried out by LC/MS/MS using blood plasma.

<Airway contraction measuring method>

Male Std:Hartley guinea pigs (Japan SLC Inc., week of age at the time of use: 6 weeks of age, from 340.6 to 488.0 g in body weight) were anesthetized with urethane, and a canula was inserted into the trachea. The trachea canula was connected to a quantitative artificial ventilator, and under artificial respiration, a catheter was indwelled in the left jugular vein. Measurement of the airway contraction was carried out by the Konzett & Rossler method. Guinea pigs were subjected to artificial respiration at a ventilation of 4 ml/animal and a ventilation frequency of 70 times/minute. Changes in the ventilation pressure were measured by a transducer (UGO BASILE, BRONCHOSPASM TRANSDUCER 7020) connected to the sub-bypass of the trachea canula. By inserting a catheter into the jugular vein, imidafenacin was administered by continuous intravenous infusion (100 $\mu$l/kg/min). After 75 minutes of the start of administration of the agent liquid, methacholine (10 $\mu$g/kg) was intravenously administered to induce airway contraction. The airway contraction inhibitory activity was evaluated using peak height of the airway contraction induced by methacholine as the index.

As a result, imidafenacin showed a dose-dependent inhibitory activity at 0.03, 0.1 and 0.3 $\mu$g/kg/min, and the $ID_{50}$ value was 0.061 $\mu$g/kg/min. Additionally, the blood concentration at around this $ID_{50}$ value was about 1 ng/ml.

In this connection, the blood concentration measurement was carried out by LC/MS/MS using blood plasma.

Namely, it was confirmed that imidafenacin Plaster and Pressure Sensitive Adhesives are useful for not only OAB but also COPD when they are pharmaceutical preparations which can keep similar degree of blood concentration.

Test Example 8: Airway contraction inhibitory effect

An adhesive plaster was prepared by dispersing imidafenacin (20 mg), SIS (790 mg), an ultra hypochromic rosin ester (ester gum) (990 mg) and isopropyl myristate (200 mg) in chloroform (7.5 g). The adhesive plaster was spread on a release liner (Scotch Pack 9742, manufactured by 3M Health Care) to be thickness of about 125 $\mu$m using an applicator. The adhesive plaster surface was dried for 20 minutes with hot air (about 50 to 60°C). By covering the thus dried adhesive plaster surface with the release liner and cutting it into a square shape of 3.2 cm $\times$ 3.2 cm (10 cm$^2$), an adhesive single layer type percutaneous absorption type pharmaceutical preparation (1 mg/10 cm$^2$) was prepared.

Under GOI (oxygen:laughing gas =3:1, 3% isoflurane) anesthesia, an abdominal region of each guinea pig was shaved. The adhesive single layer type percutaneous absorption type pharmaceutical preparation produced in the above was applied to the abdominal region and fixed with a surgical tape. After 24 hours, each of the guinea pigs of non-treated group and stuck group was anesthetized with pentobarbital sodium and then incised, followed by attaching polyethylene capillary thereto as a trachea canula. The trachea canula was connected to a quantitative artificial ventilator (Model SN-480-7, manufactured by Shinano Seisakusho), and artificial ventilation was carried out at a ventilation amount of 4 ml and a ventilation frequency of 70 times/minute. In order to administer gallamine and methacholine, a polyethylene tube filled with physiological saline was inserted into the left jugular vein. The airway contraction reaction was measured by the Konzett & Rossler method. Changes in the ventilating pressure were measured by a transducer (BRONCHOSPASM TRANSDUCER, Cat. No. 7020, UGO BASILE) connected to the sub-bypass of the artificial ventilation circuit and recorded by a recorder (LINEARCORDER, WR3320, manufactured by Graphtec Corporation).

Before inducing the airway contraction reaction, gallamine (10 mg/ml/kg) was intravenously administered. After confirming that base line of the airway contraction reaction was stabilized, the airway contraction reaction was induced by intravenously administering of methacholine (10 $\mu$g/ml/kg). As a result, the 24 hours applied group completely inhibited the methacholine-induced airway contraction. The result shows that this pharmaceutical preparation is effective for COPD.

Based on the aforementioned Test Example, the percutaneous absorption type pharmaceutical preparations to be used in the present invention are pharmaceutical preparations, which significantly improve the low skin permeability of imidafenacin; show very weak skin irritation; and have superior sustained release property. Additionally, it was confirmed also that the percutaneous absorption type pharmaceutical preparations of the present invention have persistent choline-induced airway contraction and bladder contraction inhibitory activities, and their effects are strong.

Test Example 9: Stability Test

The fact that the pharmaceutical preparations of the present invention are stable was confirmed by the following test.

The pharmaceutical preparations of the present invention were preserved (1) at room temperature for 6 months or (2) at 40°C under a relative humidity of 75% for 6 months, and used as the samples to be tested.

One sheet of a sample to be tested from which the liner was removed was put into a test tube and mixed with diethyl ether (18 ml) and an internal standard solution, followed by shaking for 15 minutes. After taking out 10 ml portion of this solution, the solvent was evaporated under a reduced pressure. Heptane (10 ml) was added to the residue and shaken for 15 minutes. A phosphoric acid-acetonitrile mixed liquid (7:3, 10 ml) was added to the solution and shaken for 15 minutes. This mixed liquid was centrifuged (2500 rpm, 10 minutes), and the lower layer was collected. The phosphoric acid-acetonitrile mixed liquid (7:3, 10 ml) was again added to the residual liquid and shaken for 15 minutes. The mixed liquid was centrifuged (2500 rpm, 10 minutes). The resulting lower layer was collected and combined with the aforementioned lower layer, and the volume was adjusted precisely to 25 ml by adding the phosphoric acid-acetonitrile mixed liquid (7:3) to obtain sample solution.

By a liquid chromatography, a 50 μl portion of the sample solution was analyzed, and the amount of imidafenacin was calculated from the ratio of the peak area of imidafenacin to the peak area of the internal standard substance in accordance with a calibration curve.

As a result, it was found that the pharmaceutical preparations of the present invention are stable pharmaceutical preparations. For example, when the pharmaceutical preparation produced in Example 7(3) was preserved under the aforementioned conditions (1) and (2), periodical change in the imidafenacin content was not found.

Industrial Applicability

The present invention provides a percutaneous absorption type pharmaceutical preparation which enables absorption of imidafenacin which has a low ability to be absorbed from the skin, from the skin into the body continuously and efficiently. Thus, the percutaneous absorption pharmaceutical preparation shows improvement in persistency of effects; avoidance of side effects; and convenience of administration, in comparison with oral administration. It is useful for not only overactive bladder (OAB) but also chronic obstructive pulmonary disease (COPD).

Brief Description of the Drawings

Fig. 1 shows results of a skin permeation test of the pharmaceutical preparations produced in Examples 1, 2 and 3(1) to 3(13), using male hairless rats.
Fig. 2 shows results of a skin permeation test of the pharmaceutical preparations produced in Examples 4(6) to 4 (10), using male hairless rats.
Fig. 3 shows correlation between the cumulative permeation amount using minipig percutaneous absorption and the cumulative permeation amount using hairless rat percutaneous absorption of the pharmaceutical preparations produced in Reference Example 1, Example 3(11) and Example 2.

**Claims**

1. A percutaneous absorption type pharmaceutical preparation which comprises 4-(2-methyl-1-imidazolyl)-2,2-diphenylbutylamide as the active ingredient and a backbone for transdermal system, and satisfies at least one of the following conditions (1) and (2):

   (1) the active ingredient content in one preparation or one time administration preparation is from about 0.1 mg to about 10 mg,
   (2) the size is from about 1 cm$^2$ to about 300 cm$^2$.

2. The pharmaceutical preparation according to claim 1, wherein the backbone for transdermal system comprises (i) one or more adhesive(s) selected from a styrene-isoprene-styrene block copolymer, a silicone rubber, a polyisobutylene rubber, a rosin resin, an polyacrylate and an alicyclic saturated hydrocarbon resin and (ii) an amphipathic solubilizing agent, a percutaneous permeation accelerator and/or a skin irritation alleviating agent.

3. The pharmaceutical preparation according to claim 2, wherein the backbone for transdermal system consists of (i) one or more adhesive(s) selected from a styrene-isoprene-styrene block copolymer, a silicone rubber, a polyisobutylene rubber, a rosin resin and an alicyclic saturated hydrocarbon resin, (ii) an amphipathic solubilizing agent and (iii) a percutaneous permeation accelerator.

4. The pharmaceutical preparation according to claim 2, wherein (i) the adhesive is one or more adhesive(s) selected from a styrene-isoprene-styrene block copolymer, a silicone rubber, an polyacrylate, a polyisobutylene rubber, a rosin resin and an alicyclic saturated hydrocarbon resin and (ii) the amphipathic solubilizing agent is one or more agent(s) selected from N-methyl-2-pyrrolidone, isopropyl myristate, propylene glycol, triacetin, benzyl alcohol, oleyl alcohol, oleic acid, diethyl sebacate, diisopropyl adipate, liquid paraffin and cetyl lactate, the percutaneous permeation accelerator is one or more accelerator(s) selected from triacetin, Crotamiton, cetyl lactate, diisopropyl adipate, oleic acid and oleyl alcohol, and the skin irritation alleviating agent is Crotamiton.

5. The pharmaceutical preparation according to claim 4, wherein the adhesive is (i) a combination of a styrene-isoprene-styrene block copolymer and a rosin resin; (ii) a silicone rubber; (iii) a combination of a silicone rubber and a rosin resin; (iv) a combination of a silicone rubber, a rosin resin and an alicyclic saturated hydrocarbon resin; (v) an polyacrylate; (vi) a combination of an polyacrylate and a silicone rubber; or (vii) a combination of a styrene-isoprene-styrene block copolymer, a polyisobutylene rubber, a rosin resin and an alicyclic saturated hydrocarbon resin.

6. The pharmaceutical preparation according to claim 2, wherein (i) the adhesive is a combination of a styrene-isoprene-styrene block copolymer and a rosin resin, and (ii-1) the amphipathic solubilizing agent is N-methyl-2-pyrrolidone, propylene glycol, triacetin, oleyl alcohol, oleic acid or cetyl lactate; (ii-2) the percutaneous permeation accelerator is triacetin, Crotamiton, cetyl lactate, oleic acid or oleyl alcohol; or (ii-3) the combination of an amphipathic solubilizing agent and a percutaneous permeation accelerator is oleic acid and Crotamiton, oleyl alcohol and Crotamiton, cetyl lactate and Crotamiton or triacetin and Crotamiton.

7. The pharmaceutical preparation according to claim 2, wherein (i) the adhesive is a silicone rubber, and (ii-1) the amphipathic solubilizing agent is N-methyl-2-pyrrolidone, isopropyl myristate, propylene glycol, triacetin, oleyl alcohol, oleic acid or cetyl lactate; (ii-2) the percutaneous permeation accelerator is triacetin, Crotamiton, cetyl lactate, oleic acid or oleyl alcohol; or (ii-3) the combination of an amphipathic solubilizing agent and a percutaneous permeation accelerator is oleic acid and Crotamiton or oleyl alcohol and Crotamiton.

8. The pharmaceutical preparation according to claim 2, wherein (i) the adhesive is a combination of a styrene-isoprene-styrene block copolymer, a polyisobutylene rubber, a rosin resin and an alicyclic saturated hydrocarbon resin, and (ii-1) the amphipathic solubilizing agent is one or more agent(s) selected from N-methyl-2-pyrrolidone, isopropyl myristate, propylene glycol, triacetin, oleyl alcohol, oleic acid, liquid paraffin and cetyl lactate; the percutaneous permeation accelerator is at least one or more accelerator(s) selected from triacetin, Crotamiton, cetyl lactate, oleic acid and oleyl alcohol; and the skin irritation alleviating agent is Crotamiton.

9. The pharmaceutical preparation according to claim 2, wherein based on 1 part by mass of the active ingredient, the adhesive is from about 25 parts by mass to about 350 parts by mass; and (i) the amphipathic solubilizing agent and/or percutaneous permeation accelerator is from about 2 parts by mass to about 400 parts by mass or (ii) the amphipathic solubilizing agent and/or percutaneous permeation accelerator is from about 2 parts by mass to about 200 parts by mass; and the skin irritation alleviating agent is from about 0.1 part by mass to about 10 parts by mass.

10. The pharmaceutical preparation according to claim 3, wherein the amphipathic solubilizing agent is (i) liquid paraffin and (ii) N-methyl-2-pyrrolidone and/or propylene glycol, and the percutaneous permeation accelerator is oleic acid.

11. The pharmaceutical preparation described in claim 3, wherein based on 1 part by mass of the active ingredient, the adhesive is from about 25 parts by mass to about 350 parts by mass; the amphipathic solubilizing agent is from about 2 parts by mass to about 400 parts by mass; and the percutaneous permeation accelerator is from about 2 parts by mass to about 200 parts by mass.

12. The pharmaceutical preparation according to any one of claims 2 to 11, which further comprises from about 0.01 part by weight to about 10 parts by weight of dibutylhydroxytoluene or DL-$\alpha$-tocopherol, based on 1 part by weight of the active ingredient.

13. The pharmaceutical preparation according to claim 1, which comprises 4-(2-methyl-1-imidazolyl)-2,2-diphenylbutylamide, which is a soluble type or a mixed type of soluble type and non-soluble type as the active ingredient.

14. The pharmaceutical preparation according to any one of claims 1 to 13, wherein blood concentration of 4-(2-metlayl-1-imidazolyl)-2,2-diphenylbutylamide is maintained in a range of from about 10 pg/ml to about 10 ng/ml for about 0.5 day to about 2 days after its administration.

15. The pharmaceutical preparation according to claim 1, which is a Plaster and Pressure Sensitive Adhesive.

16. The pharmaceutical preparation according to claim 15, which has an adhesive plaster having a thickness of from about 10 $\mu$m to about 2000 $\mu$m.

**17.** The pharmaceutical preparation according to claim 15, which is a plaster or a cataplasm.

**18.** The pharmaceutical preparation according to claim 1, which is an agent for preventing and/or treating a disease selected from pollakiurea and urinary incontinence accompanied by over active bladder, asthma, chronic obstructive pulmonary disease and irritable bowel syndrome.

**19.** A Plaster and Pressure Sensitive Adhesive, which uses 4-(2-methyl-1-imidazolyl)-2,2-diphenylbutylamide as an active ingredient, which comprises (i) an adhesive consisting of a combination of a styrene-isoprene-styrene block copolymer, a polyisobutylene rubber, a rosin resin and an alicyclic saturated hydrocarbon resin, which is from about 25 parts by mass to about 160 parts by mass, based on 1 part by mass of the active ingredient; (ii) liquid paraffin which is from about 20 parts by mass to about 80 parts by mass, based on 1 part by mass of the active ingredient; (iii) N-methyl-2-pyrrolidone and/or propylene glycol which is from about 2 parts by mass to about 100 parts by mass, based on 1 part by mass of the active ingredient; and (iv) oleic acid which is from about 2 parts by mass to about 50 parts by mass, based on 1 part by mass of the active ingredient, and which comprises (v) can maintain blood concentration of the active ingredient within a range of from about 10 pg/ml to about 3 ng/ml for about 0.5 day to about 2 days after its administration, wherein it satisfies at least one of the following conditions (1) to (3):

(1) the active ingredient content in one preparation or one time administration preparation is from about 0.1 mg to about 2 mg,
(2) the size is from about 1 cm$^2$ to about 40 cm$^2$,
(3) thickness of the adhesive plaster is from about 20 $\mu$m to about 200 $\mu$m.

## FIG. 1

## FIG. 2

## FIG. 3

Plot with x-axis "CUMULATIVE PERMEATION AMOUNT (μg/cm²) OF MINIPIGS AFTER 34 HOURS" ranging 0 to 12, and y-axis "CUMULATIVE PERMEATION AMOUNT (μg/cm²) OF HAIRLESS RATS AFTER 26 HOURS" ranging 0 to 30.

$$y = 1.4595x + 9.1409$$
$$R^2 = 0.975$$

<table>
<tr><td colspan="2" align="center">**INTERNATIONAL SEARCH REPORT**</td><td colspan="2">International application No.<br>PCT/JP2006/301759</td></tr>
</table>

A.  CLASSIFICATION OF SUBJECT MATTER
*A61K31/4164*(2006.01), *A61K9/70*(2006.01), *A61K47/06*(2006.01), *A61K47/10*
(2006.01), *A61K47/12*(2006.01), *A61K47/14*(2006.01), *A61K47/16*(2006.01),
*A61K47/22*(2006.01), *A61K47/32*(2006.01), *A61K47/46*(2006.01), *A61P1/00*(2006.01),
According to International Patent Classification (IPC) or to both national classification and IPC

B.  FIELDS SEARCHED

Minimum documentation searched (classification system followed by classification symbols)
A61K9/70,A61K31/4164,A61K47/06,A61K47/10,A61K47/12,A61K47/14,A61K47/16,
A61K47/22, A61K47/32, A61K47/46, A61P1/00, A61P11/00, A61P11/06, A61P13/02

Documentation searched other than minimum documentation to the extent that such documents are included in the fields searched
Jitsuyo Shinan Koho          1922-1996   Jitsuyo Shinan Toroku Koho   1996-2006
Kokai Jitsuyo Shinan Koho    1971-2006   Toroku Jitsuyo Shinan Koho   1994-2006

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
|---|---|---|
| Y | JP 7-215943 A  (Kyorin Pharmaceutical Co., Ltd.),<br>15 August, 1995 (15.08.95),<br>Full text<br>& WO 95/015951 A1          & EP 733621 A1<br>& EP 733621 A4          & US 5932607 A<br>& US 6103747 A          & EP 733621 B1<br>& JP 3294961 B2 | 1-19 |
| Y | WO 2000/064435 A1  (Lead Chemical Co., Ltd.),<br>02 November, 2000 (02.11.00),<br>Full text<br>& EP 1174132 A1          & JP 2000-613426 A | 1-19 |

☒  Further documents are listed in the continuation of Box C.          ☐  See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>27 April, 2006 (27.04.06) | Date of mailing of the international search report<br>16 May, 2006 (16.05.06) |
|---|---|
| Name and mailing address of the ISA/<br>Japanese Patent Office | Authorized officer |
| Facsimile No. | Telephone No. |

Form PCT/ISA/210 (second sheet) (April 2005)

**INTERNATIONAL SEARCH REPORT**

International application No.

PCT/JP2006/301759

C (Continuation). DOCUMENTS CONSIDERED TO BE RELEVANT

| Category* | Citation of document, with indication, where appropriate, of the relevant passages | Relevant to claim No. |
|---|---|---|
| Y | JP 10-152434 A (Nitto Denko Corp.),<br>09 June, 1998 (09.06.98),<br>Full text<br>& WO 98/013035 A1 | 1-19 |
| Y | WO 95/031190 A1 (Hisamitsu Pharmaceutical Co., Inc.),<br>23 November, 1995 (23.11.95),<br>Full text<br>& JP 7-529521 A  & EP 760238 A1<br>& US 5770221 A  & EP 760238 B1 | 1-19 |
| Y | JP 2001-039873 A (Nichiban Co., Ltd.),<br>13 February, 2001 (13.02.01),<br>Full text<br>(Family: none) | 1-19 |
| Y | JP 6-145052 A (Hisamitsu Pharmaceutical Co., Inc.),<br>24 May, 1994 (24.05.94),<br>Full text<br>(Family: none) | 1-19 |
| Y | JP 4-266821 A (Lead Chemical Co., Ltd.),<br>22 September, 1992 (22.09.92),<br>Full text<br>(Family: none) | 1-19 |
| Y | JP 4-273818 A (Kissei Pharmaceutical Co., Ltd.),<br>30 September, 1992 (30.09.92),<br>Full text<br>(Family: none) | 1-19 |

Form PCT/ISA/210 (continuation of second sheet) (April 2005)

**INTERNATIONAL SEARCH REPORT**

| International application No. |
|---|
| PCT/JP2006/301759 |

Continuation of A. CLASSIFICATION OF SUBJECT MATTER
(International Patent Classification (IPC))

*A61P11/00*(2006.01), *A61P11/06*(2006.01), *A61P13/02*(2006.01)

(According to International Patent Classification (IPC) or to both national classification and IPC)

Form PCT/ISA/210 (extra sheet) (April 2005)

## REFERENCES CITED IN THE DESCRIPTION

### Patent documents cited in the description

- JP 7215943 A
- WO 0134147 A
- WO 01134147 A

### Non-patent literature cited in the description

- *Bioorg. Med. Chem.,* 1999, vol. 7, 1151-1161
- *J. Pharmacal. Exp. Ther.,* 1944, vol. 83, 377-390